# EUROPÄISCHE PATENTSCHRIFT

(11) **EP 1 156 715 B1**
(45) Veröffentlichungstag und Bekanntmachung des Hinweises auf die Patenterteilung: **07.05.2003**
(21) Anmeldenummer: 00912492.6
(22) Anmeldetag: 21.02.2000
(51) Int. Cl.: A01N 37/34, C07C 255/41

(54) **VERWENDUNG VON PHENYLESSIGSAÜREAMIDE ALS PFLANZENSCHUTZMITTEL MIT HERBIZIDER UND FUNGIZIDER WIRKUNG**
USE OF PHENYLACETAMIDES AS PLANT PROTECTIVES HAVING HERBICIDAL AND FUNGICIDAL EFFECT
UTILISATION D'AMIDES D'ACIDE PHENYLACETIQUE EN TANT QUE PRODUITS PHYTOSANITAIRES A EFFET HERBICIDE ET FONGICIDE

(30) Priorität: 02.03.1999 DE 19909037
(43) Veröffentlichungstag der Anmeldung: 28.11.2001
(73) Patentinhaber: BASF AKTIENGESELLSCHAFT, 67056 Ludwigshafen (DE)
(72) Erfinder: WAGNER, Oliver, D-67433 Neustadt (DE); GROTE, Thomas, D-67157 Wachenheim (DE); AMMERMANN, Eberhard, D-64646 Heppenheim (DE); STRATHMANN, Siegfried, D-67117 Limburgerhof (DE); LORENZ, Gisela, D-67434 Neustadt (DE); GROSSMANN, Klaus, D-67141 Neuhofen (DE)
(74) Vertreter: Kinzebach, Werner, Dr.
(86) Internationale Anmeldenummer: EP0001409
(87) Internationale Veröffentlichungsnummer: WO00051431

(56) Entgegenhaltungen:
- EP-A- 0 466 640
- WO-A-95/35283
- DD-A- 156 663
- DE-A- 2 008 692
- FR-A- 2 491 061
- A. D. WHITE: "Heterocyclic amides: inhibitors of Acyl-CoA: Cholesterol O-acyltransferase with hypocholesterolemic activity in several species and antiatherosclerotic activity in the rabbit" J.MED.CHEM., Bd. 39, 1996, Seiten 3908-3916, XP002137544 in der Anmeldung erwähnt
- GERMAIN ET AL.: "Synthèse d'indoles par cyclisation réductrice. II. (1) nouvelle méthode de synthèse des indole carboxamides-3, donnant accès aux dérivés de la gramine" J. HETEREOCYCLIC CHEM., Bd. 13, 1976, Seiten 1209-1218, XP002137545
- BOURDAIS ET AL.: "Une synthèse directe des indole-carboxamides-3: nouvel accès aux dérivés de la gramine" TETRAHEDRON LETTERS, Nr. 3, 1970, Seiten 195-198, XP002137546
- CHEMICAL ABSTRACTS, vol. 110, no. 1, 1989 Columbus, Ohio, US; abstract no. 192, KNABE ET AL.: "Acylanilides. III. Formation of methemoglobin and its metabolismin rats" XP002137547 & ARCH. PHARM., Bd. 321, Nr. 10, - 1988 Seiten 739-741, in der Anmeldung erwähnt
- CHEMICAL ABSTRACTS, vol. 109, no. 5, 1988 Columbus, Ohio, US; abstract no. 37587, KNABE ET AL.: "Acylanilides. I. Syntheses of the racemates and enantiomers of chiral acylanilides" XP002137548 & ARCH.PHARM. , Bd. 321, Nr. 2, 1988, Seite 107-110 in der Anmeldung erwähnt

## Beschreibung

Die vorliegende Erfindung betrifft die Verwendung von Phenylessigsäureamiden als Pflanzenschutzmittel, neue Pflanzenschutzmittel enthaltend als Wirkstoffe Phenylessigsäureamide und neue Phenylessigsäureamide.

Gegenstand der vorliegenden Erfindung ist die Verwendung von Phenylessigsäureamiden der Formel I 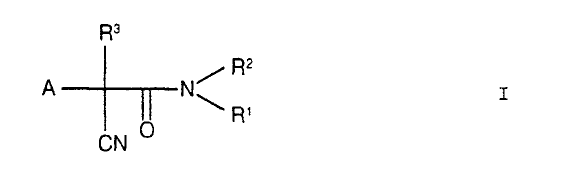 als Pflanzenschutzmittel, wobei die Reste folgende Bedeutungen haben:
- A: Aryl, das durch folgende Gruppen ein- oder mehrfach substituiert sein kann: Wasserstoff, Halogen, Cyano, Nitro, Hydroxy, Mercapto, Thiocyanato, Carboxy, Amino, C₁-C₆-Alkyl, C₁-C₆-Alkoxy-C₁-C₆-Alkyl, C₁-C₆-Cyanoalkyl, C₂-C₆-Alkenyl, C₁-C₆-Alkoxy-C₂-C₆-Alkenyl, C₂-C₆-Alkinyl, C₁-C₆-Alkoxy-C₃-C₆-Alkinyl, C₃-C₆-Alkinyl-C₁-C₆-Alkoxy, C₃-C₇-Cycloalkyl, C₁-C₆-Alkyl-C₃-C₇-Cycloalkyl, C₁-C₆-Alkoxy-C₃-C₇-Cycloalkyl, C₃-C₇-Cycloalkyloxy, C₃-C₇-Cyanocycloalkoxy, C₁-C₆-Alkyl-C₃-C₇-Cycloalkoxy, C₃-C₇-Halogencycloalkyl, C₃-C₇-Cyanocycloalkyl, C₃-C₇-Halogencycloalkoxy, C₅-C₇-Cycloalkenyl, C₁-C₆-Alkyl-C₅-C₇-Cycloalkenyl, C₁-C₆-Alkoxy-C₅-C₇-Cycloalkenyl, C₅-C₇-Cyanocycloalkenyl, C₅-C₇-Halogencycloalkenyl, C₁-C₆-Halogenalkyl, C₂-C₆-Halogenalkenyl, C₃-C₆-Halogenalkinyl, C₂-C₆-Cyanoalkenyl, C₃-C₆-Cyanoalkinyl, C₁-C₆-Alkoxy, C₁-C₆-Cyanoalkoxy, C₁-C₆-Halogenalkoxy, C₂-C₆-Alkenyloxy, C₂-C₆-Cyanalkenyloxy, C₂-C₆-Halogenalkenyloxy, C₂-C₆-Alkinyloxy, C₁-C₆-Alkylthio, C₁-C₆-Cyanoalkylthio, C₁-C₆-Halogenalkylthio, C₁-C₆-Alkoxy- C₁-C₆-Alkylthio, C₁-C₆-Alkoxycarbonyl, C₁-C₆-Alkylcarbonyl, C₁-C₆-Alkylcarboxy-C₁-C₆-Alkyl, C₁-C₆-Alkylcarbonyl-C₁-C₆-Alkyl, C₁-C₆-Alkylcarbonylamino, Arylcarbonylamino, Arylsulphinyl, Arylsulphonyl, Heteroarylsulphinyl, Heteroarylsulphonyl, Aryl-C₁-C₆-Alkyl, Aryl-C₂-C₆-Alkenyl, Aryl-C₃-C₆-Alkinyl, Aryl-C₁-C₆-Alkoxy, Aryl-C₂-C₆-Alkenyloxy, Aryl-C₃-C₆-Alkinyloxy, C₁-C₆-Alkylsulphinyl, C₁-C₆-Alkylsulphonyl, C₁-C₆-Alkylamino, di-C₁-C₆-Alkylamino, Aryl, Aryl, Aryloxy, Arylthio oder -C(R⁴)=NR⁵,
- R¹: Aryl oder Aryl-C₁-C₆-alkyl, wobei der Arylrest jeweils durch folgende Gruppen ein- oder mehrfach substituiert sein kann: Wasserstoff, Halogen, Cyano, Nitro, Hydroxy, Mercapto, Thiocyanato, Carboxy, Amino, C₁-C₆-Alkyl, C₁-C₆-Alkoxy-C₁-C₆-Alkyl, C₁-C₆-Cyanoalkyl, C₂-C₆-Alkenyl, C₁-C₆-Alkoxy-C₂-C₆-Alkenyl, C₂-C₆-Alkinyl, C₁-C₆-Alkoxy-C₃-C₆-Alkinyl, C₃-C₆-Alkinyl-C₁-C₆-Alkoxy, C₃-C₇-Cycloalkyl, C₁-C₆-Alkyl-C₃-C₇-Cycloalkyl, C₁-C₆-Alkoxy-C₃-C₇-Cycloalkyl, C₃-C₇-Cycloalkyloxy, C₃-C₇-Cyanocycloalkoxy, C₁-C₆-Alkyl-C₃-C₇-Cycloalkoxy, C₃-C₇-Halogencycloalkyl, C₃-C₇-Cyanocycloalkyl, C₃-C₇-Halogencycloalkoxy, C₅-C₇-Cycloalkenyl, C₁-C₆-Alkyl-C₅-C₇-Cycloalkenyl, C₁-C₆-Alkoxy-C₅-C₇-Cycloalkenyl, C₅-C₇-Cyanocycloalkenyl, C₅-C₇-Halogencycloalkenyl, C₁-C₆-Halogenalkyl, C₂-C₆-Halogenaikenyl, C₃-C₆-Halogenalkinyl, C₂-C₆-Cyanoalkenyl, C₃-C₆-Cyanoalkinyl, C₁-C₆-Alkoxy, C₁-C₆-Cyanoalkoxy, C₁-C₆-Halogenalkoxy, C₂-C₆-Alkenyloxy, C₂-C₆-Cyanalkenyloxy, C₂-C₆-Halogenalkenyloxy, C₂-C₆-Alkinyloxy, C₁-C₆-Alkylthio, C₁-C₆-Cyanoalkylthio, C₁-C₆-Halogenalkylthio, C₁-C₆-Alkoxy-C₁-C₆-Alkylthio, C₁-C₆-Alkoxycarbonyl, C₁-C₆-Alkylcarbonyl, C₁-C₆-Alkylcarboxy-C₁-C₆-Alkyl, C₁-C₆-Alkylcarbonyl-C₁-C₆-Alkyl, C₁-C₆-Alkylcarbonylamino, Arylcarbonylamino, Arylsulphinyl, Arylsulphonyl, Heteroarylsulphinyl, Heteroarylsulphonyl, Aryl-C₁-C₆-Alkyl, Aryl-C₂-C₆-Alkenyl, Aryl-C₃-C₆-Alkinyl, Aryl-C₁-C₆-Alkoxy, Aryl-C₂-C₆-Alkenyloxy, Aryl-C₃-C₆-Alkinyloxy, C₁-C₆-Alkylsulphinyl, C₁-C₆-Alkylsulphonyl, C₁-C₆-Alkylamino, di-C₁-C₆-Alkylamino, Aryl, Aryl, Aryloxy, Arylthio oder -C(R⁴)=NR⁵,
- R², R³: Wasserstoff, C₁-C₆-Alkyl, C₂-C₆-Alkenyl, C₃-C₇-Cycloalkyl, C₁-C₆-Alkylaryl, welche ggf. teilweise oder vollständig halogeniert sein können oder einen bis drei Substituenten ausgewählt aus C₁-C₄-Alkoxy, C₁-C₄-Halogenalkoxy, C₁-C₄-Alkylthio, C₃-C₇-Cycloalkyl, C₅-C₇-Cycloalkenyl tragen können;
- R⁴: Wasserstoff oder C₁-C₆-Alkyl,
- R⁵: Hydroxy, C₁-C₄-Alkoxy, Aryl-C₁-C₄-Alkoxy, Aryloxy,
sowie deren landwirtschaftlich brauchbaren Salze.

Einige der Verbindungen der Formel I sind literaturbekannt.

In WO 95/35283 werden trisubstituierte Phenylderivate als Arzneimittel beschrieben, die als PDE IV (Phosphodiesterase IV) Inhibitoren fungieren. Insbesondere werden dort Verbindungen der folgenden Formel 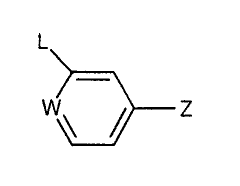 beschrieben, wobei unter anderem W die Gruppe =C(Y)- darstellt, mit Y = Halogen, Alkyl, -X-R^{a} oder -N(R^{b}), wobei X = O, S, SO, S; R^{a} = Wasserstoff, Alkyl; R^{b} = Wasserstoff oder Alkyl; L = -CH(R¹) (R²) mit R¹ bzw. R² = CN oder -CONR⁹R¹⁰ und R⁹ bzw. R¹⁰ = Wasserstoff, Alkyl, Aralkyl oder Aryl; Z = eine der dort näher bezeichneten Gruppen (A) - (D), wobei diese Gruppen obligatorisch durch mindestens eine Arylgruppe (Ar) substituiert sind.

In EP 0 466 640 werden Alpha-Carbonylphenylacetonitril-Derivate als Stabilisatoren für organische Materialien beschrieben. Die dort offenbarten Verbindungen der Formel 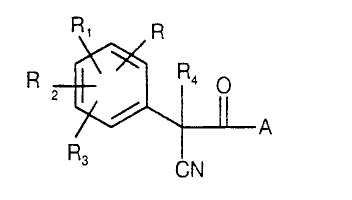 eignen sich zur Stabilisierung von Schmierölen, Metallbearbeitungsflüssigkeiten, Hydraulikflüssigkeiten sowie von thermoplastischen Kunststoffen und Elastomeren. In der oben angegebenen Formel bedeutet A unter anderem die Gruppe -NR⁶R⁷, wobei R⁶, R⁷ unter anderem Wasserstoff und unsubstituiertes Phenyl oder Naphthyl bedeuten können. Zwei zueinander in ortho-Stellung gebundene Reste R, R¹, R², R³ oder R⁴ können auch die Gruppe -CH=CH-CH=CH-(Naphthylgruppe) bilden.

In DD 156,663 werden Verbindungen der folgenden Formel als pflanzenwachstumsstimulierende Mittel zur Anwendung bei Kulturpflanzen, wie z.B. Mais, Sonnenblume, Tomate, Soja oder Bohnen, beschrieben:

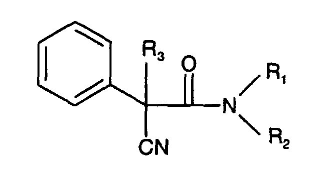

Die Reste haben dort unter anderem folgende Bedeutung: R₁, R₂: Wasserstoff, Alkyl, Cycloalkyl, Aryl, Halogenaryl, Aralkyl, Alkenyl oder Alkanoyl; R₃: Wasserstoff oder Alkyl. Die beschriebenen Verbindungen zeichnen sich dadurch aus, daß die Phenylgruppe in jedem Fall unsubstituiert ist.

Die FR 2,491,061 beschreibt Pflanzenwuchsregulatoren der Formel, 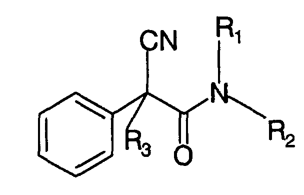 worin R₁ und R₂ u.a. für Wasserstoff, C₁-C₅-Alkyl, C₅-C₇-Cycloalkyl, Aryl, halogen-substituiertes Aryl, Aralkyl, C₂-C₅-Alkenyl oder C₂-C₅-Alkinyl stehen und R₃ für Wasserstoff oder C₁-C₅-Alkyl steht. Die Phenylgruppe ist stets unsubstituiert.

In DE 2 008 692 werden Cyanphenylessigsäureamidderivate der folgenden Formel 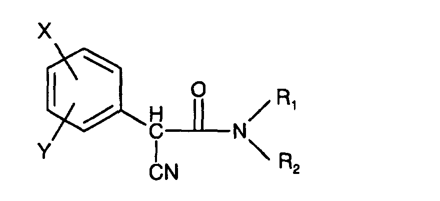 als Ausgangsstoffe bzw. Zwischenprodukte zur Herstellung von Indol-3-carbonsäurederivaten beschrieben. R₁ bzw. R₂ bedeuten dort Wasserstoff, Alkyl oder Aralkyl. Die beiden Reste X und Y am Phenylring sind u.a. Wasserstoff, Halogen, Alkyl, Alkoxy, Aralkoxy, Trifluormethyl, Hydroxy, Amino, Nitro, Carboxy oder Sulfoxy. Zusätzlich kann der Phenylring durch eine Amino- oder Nitrogruppe substituiert sein.

Weiterhin sind folgende Einzelverbindungen der Formel I aus der wissenschaftlichen Literatur bekannt:
a) Die Verbindung mit A=Phenyl, R¹=4-Ethoxyphenyl, R²=H, R³=n-[2-Cyano-2-phenyl-hexansäure-(N-4-ethoxyphenyl)amid] aus Aren. Pharm. 1988, 321: 107.
b) Die Verbindung mit A=Phenyl, R¹=4-Ethoxyphenyl, R²=H, R³=C₂H₅; [2-Cyano-2-phenyl-butansäure-(N-4-ethoxyphenyl)amid] aus Arch. Pharm. 1988, 321: 739.
c) Die Verbindung mit A=Phenyl, R¹=2,4,6-Trimethoxyphenyl, R²=H, R³=H; [2-Cyano-2-phenyl-essigsäure-(N-2,4,6-trimethoxyphenyl)amid] aus J. Med. Chem. 1996, 39,20: 3908.
d) Die Verbindung mit A=4-Methoxyphenyl, R¹=2,6-Dimethylphenyl, R²=H, R³=H; [2-Cyano-2-(4-methoxyphenyl)essigsäure-(N-2,6-dimethylphenyl)amid] aus Liebigs Ann. Chem. 1992, 3: 239.
e) Die Verbindungen mit A=Phenyl, R¹=2-Methylyphenyl, 3-Methylphenyl oder 4-Methylphenyl; R²=H, R³=H; [2-Cyano-2-phenylessigsäure-(N-2-methylphenyl)amid; 2-Cyano-2-phenylessigsäure-(N-3-methylphenyl)amid; 2-Cyano-2-phenylessigsäure-(N-4-methylphenyl)amid] aus J. Am. Chem. 39, 1908, 76.
f) Die Verbindung mit A=2-Nitro-4-trifluormethylphenyl, R¹=Phenyl, R²=H; [2-Cyano-2-(2-nitro-4-trifluormethylphenyl)essigsäure-N-phenylamid], aus Heterocyclic. Chem., Bd. 13, 1976, S. 1209 -1218.
g) Die Verbindung mit A=2-Amino-4-trifluormethylphenyl, R¹=Phenyl, R²=H [2-Cyano-2-(2-amino-4-trifluormethylphenyl)essigsäure-N-phenylamid] aus Tetrahedron Letters No. 3, SS. 195-198, 1970.

Überraschenderweise wurde gefunden, daß Verbindungen der Formel I als Pflanzenschutzmittel mit fungizider und/oder herbizider Wirkung verwendet werden können. Sie eignen sich zur Behandlung von Pflanzen, beispielsweise zur Bekämpfung von Schadpilzen bei Nutzpflanzen (fungizide Wirkung) oder zur Bekämpfung des unerwünschten Pflanzenwuchses bei Schadpflanzen (herbizide Wirkung).

Verbindungen der Formel I lassen sich analog zu den aus der Literatur bekannten Verfahren (z.B. J. Am. Chem. 39, 1908, 63; DD 156663) herstellen. Die Ausgangsstoffe sind entweder literaturbekannt bzw. können analog zu literaturbekannten Methoden hergestellt werden oder sind kommerziell erhältlich.

Gegenstand der vorliegenden Erfindung sind ferner Pflanzenschutzmittel, enthaltend im Gemisch mit Hilfsstoffen, die die Ausbringung des Mittels auf Freiflächen oder zur Behandlung von Pflanzen ermöglichen, mindestens eine der Verbindungen der in Anspruch 1 angegebenen Formel I, worin die Reste die in einem der Ansprüche 1 bis 11 angegebene Bedeutung haben, mit Ausnahme der Verbindungen, in denen A eine unsubstituierte Phenylgruppe; R³ Wasserstoff oder C₁- bis C₅-Alkyl bedeutet; und R¹ Aryl oder Aryl, das durch Halogen substituiert ist, oder Arylalkyl ist.

Gegenstand der vorliegenden Erfindung sind ferner Verbindungen der in Anspruch 1 angegebenen Formel I, worin die Reste die in einem der Ansprüche 1 bis 11 angegebene Bedeutung haben,
mit Ausnahme der Verbindungen, in denen A eine unsubstituierte Phenylgruppe; R³ Wasserstoff oder C₁- bis C₅-Alkyl bedeutet; und R¹ Aryl oder Aryl ist, das durch Halogen substituiert ist, oder Arylalkyl ist,
mit Ausnahme der Verbindungen, in denen R³ Wasserstoff ist, R¹ Phenyl, Naphthyl oder Phenyl-C₁-C₄-alkyl ist und R² Wasserstoff, Alkyl, Alkenyl oder C₅-C₇-Cycloalkyl ist,
für den Fall, daß R³ Wasserstoff ist, A nicht 2-Nitrophenyl oder 2-Aminophenyl bedeutet; und
mit Ausnahme der folgenden Verbindungen:
2-Cyano-2-phenylhexansäure-(N-4-ethoxyphenyl)amid;
2-Cyano-2-phenylbutansäure-(N-4-ethoxyphenyl)amid;
2-Cyano-2-phenylessigsäure-(N-2,4,6-trimethoxyphenyl)amid;
2-Cyano-2-(4-methoxyphenyl)essigsäure-(N-2,6-dimethylphenyl)amid;
2-Cyano-2-phenyl-essigsäure-(N-2-methylphenyl)amid;
2-Cyano-2-phenyl-essigsäure-(N-3-methylphenyl)amid;
2-Cyano-2-phenyl-essigsäure-(N-4-methylphenyl)amid;
2-Cyano-2-(2-nitro-4-trifluormethylphenyl)essigsäure-N-phenylamid;
2-Cyano-2-(2-amino-4-trifluormethylphenyl)essigsäure-N-phenylamid.

Bei der Definition der verschiedenen Reste in Formel I stehen die angegebenen Begriffe, entweder für sich allein (wie z.B.
C₁-C₆-"Alkyl") oder als Teile bzw. in Kombination mit anderen zusammengesetzten chemischen Gruppen (wie z.B. C₁-C₆-Halogen-"alkyl", Alkoxy-C₁-C₆-"alkyl"), grundsätzlich als Sammelbegriff für eine Gruppe von Verbindungen. Für den Fall, daß die genannten Gruppen ein- oder mehrfach substituiert sein können, so können die Substituenten grundsätzlich gleich oder verschieden sein.

Halogen steht jeweils für Fluor, Brom, Chlor oder Iod, insbesondere für Fluor oder Chlor.

Ferner stehen beispielsweise:
- C₁-C₄-Alkyl sowie alle Alkylteile von entsprechend zusammengesetzten chemischen Gruppen, wie z.B. Alkylamino, Dialkylamino, Alkylaryl, Cyanoalkyl, Halogenalkyl, etc.: für einen geradkettigen oder verzweigten Alkylrest, wie z.B. Methyl, Ethyl, n-Propyl, 1-Methylethyl, n-Butyl, 1-Methylpropyl, 2-Methylpropyl oder 1,1-Dimethylethyl, insbesondere für Methyl oder Ethyl;
- C₁-C₆-Alkyl: für einen geradkettigen oder verzweigten C₁-C₆-Alkylrest, wie z.B. C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₂-C₆-Alkenyl sowie alle Alkenylteile von entsprechend zusammengesetzten Gruppen, wie z.B. Alkenyloxy, Halogen-alkenyl, Halogenalkenyloxy, etc.: für einen geradkettigen oder verzweigten Alkenylrest, wie z.B. Ethylen,Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en-1-yl und 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimetyl-but-2-en-1-yl, 1,2-Dimethyl-but-3-en-1-yl, 1,3-Dimetyl-but-1-en-1-yl, 1,3-Dimethyl-but-2-en-1-yl, 1,3-Dimetyl-but-3-en-1-yl, 2,2-Dimethyl-but-3-en-1-yl, 2,3-Dimetyl-but-1-en-1-yl, 2,3-Dimethyl-but-2-en-1-yl, 2,3-Dimetyl-but-3-en-1-yl, 3,3-Dimethyl-but-1-en-1-yl, 3,3-Dimetyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;
- C₂-C₆-Alkinyl, sowie alle Alkinylteile von entsprechend zusammengesetzten chemischen Gruppen, wie z.B. Alkinyloxy oder Alkin-alkoxy, etc.: beispielsweise Ethinyl, Propargyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methyl-pent-1-in-4-yl, 3-Methylpent-1-in-5-yl, 4-Methyl-pent-2-in-4-yl oder 4-Methylpent-2-in-5-yl;
- C₁-C₆-Halogenalkyl für einen C₁-C₆-Alkylrest wie vorstehend genannt, der partiell oder vollständig durch Fluor, Chlor, Brom und/oder Iod substituiert ist, z.B. Trichlormethyl, Trifluormethyl, 2-Fluorethyl, 2-Chlorethyl, 2-Bromethyl, 2,2-Difluorethyl, 2,2,2-Trifluorethyl, 2,2,2-Trichlorethyl, 2-Fluorpropyl, 3-Fluorpropyl, 2-Chlorpropyl oder 3-Chlorpropyl, insbesondere für 2-Fluorethyl oder 2-Chlorethyl;
- C₁-C₆-Alkoxy sowie alle Alkoxyteile von entsprechend zusammengesetzten chemischen Gruppen, wie z.B. Alkoxyalkyl, Alkoxyalkenyl, Alkoxyalkinyl, Alkoxycarbonyl, etc.: für einen geradkettigen oder verzweigten Alkoxyrest, wie z.B. Methoxy, Ethoxy, Propoxy, 1-Methylethoxy, Butoxy, 1-Methylpropoxy, 2-methylpropoxy und 1,1-Dimethylethox insbesondere für Methoxy oder Ethoxy;
- Halogen-C₁-C₆-alkoxy für einen C₁-C₆-Alkoxyrest wie vorstehend genannt, der partiell oder vollständig durch Fluor, Chlor oder Brom substituiert ist;
- Halogen-C₂-C₆-alkenyl für einen C₂-C₆-Alkenylrest wie vorstehend genannt, der partiell oder vollständig durch Fluor, Chlor oder Brom substituiert ist;
- Halogen-C₂-C₆-alkinyl für einen C₂-C₆-Alkinylrest wie vorstehend genannt, der partiell oder vollständig durch Fluor, Chlor oder Brom substituiert ist;
- C₃-C₈-Cycloalkyl sowie alle Cycloalkylteile von entsprechend zusammengesetzten chemischen Gruppen, wie z.B. Cycloalkyl-alkoxy, Alkylcycloalkyl, Alkylcycloalkyloxy, Cycloalkyloxy, etc.: für Cyclopropyl, Cyclobutyl, Cyclopentyl, Cyclohexyl, Cycloheptyl, Cyclooctyl;
- C₃-C₈-Cycloalkenyl sowie alle Cycloalkenylteile von entsprechend zusammengesetzten chemischen Gruppen, wie z.B. Alkylcycloalkenyl, Cyanocycloalkenyl, etc.: Cyclopropenyl, Cyclobutenyl, Cyclopentenyl, Cyclohexenyl, Cycloheptenyl, Cyclooctenyl;
- C₁-C₆-Alkyl-carbonyl sowie alle Alkylcarbonylteile von entsprechend zusammengesetzten chemischen Gruppen, wie z.B. Alkylcarbonylalkyl, Alkylcarbonylamino, etc.: für eine Carbonylgruppe, die durch einen C₁-C₆-Alkylrest substituiert ist, wie z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylthio für ein Schwefelatom, das durch einen C₁-C₆-Alkylrest wie vorstehend genannt substituiert ist;
- C₁-C₆-Cyanoalkyl: z.B. Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyanobut-3-yl, 1-Cyano-2-methyl-prop-3-yl, 2-Cyano-2-methylprop-3-yl, 3-Cyano-2-methyl-prop-3-yl oder 2-Cyanomethylprop-2-yl;
- Aryl sowie alle Arylteile von entsprechend zusammengesetzten chemischen Gruppen, wie z.B. Aryl-C₁-C₆-alkyl, Aryl-C₂-C₆-alkenyl, Aryloxy, Arylthio, Arylcarbonylamino, etc.: ein ein- bis dreikerniges aromatisches Ringsystem enthaltend 6 - 14 Kohlenstoffring-glieder, wie z.B. Phenyl, Naphthyl und Anthracenyl;
- Aryl-C₁-C₆-alkyl: für eine geradkettige oder verzweigte C₁-C₆-Alkylgruppe, wie voranstehend genannt, wobei die Alkylgruppe durch Aryl, wie vorstehend genannt, substituiert ist; wie z.B. Arylmethyl, 2-Arylethyl, 1-Arylethyl, 3-Aryl-propyl, 2-Aryl-propyl;
- Hetaryl sowie alle Hetarylteile von entsprechend zusammengesetzten chemischen Gruppen, wie z.B. Hetarylthio, etc.: aromatische mono- oder polycyclische fünf- oder sechsgliedrige Ringsysteme, welche neben Kohlenstoff-ringgliedern zusätzlich 1 bis 4 Stickstoffatome oder 1 bis 3 Stickstoffatome und ein Sauerstoff- oder ein Schwefelatom oder ein Sauerstoff- oder ein Schwefelatom enthalten können.
- Iminogruppe -C(R⁴)=NR⁵ für z.B.: -HC=NOH, C₁-C₆-Alkyl-C=NOH, -HC=NO-C₁-C₆-Alkyl, -HC=NO-C₁-C₆-Alkylaryl, C₁-C₆-Alkyl-C=NO-C₁-C₆-Alkyl, -HC=NO-C₂-C₆-Alkenyl, C₁-C₆-Alkyl-C=NO-C₂-C₆-Alkenyl, -HC=N-O-Aryl oder C₁-C₆-Alkyl-C=N-O-Aryl.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische. Die Mengenverhältnisse können dabei in Abhängigkeit von den Gruppen unterschiedlich sein. Die Gemische können ggf. nach den üblichen Methoden getrennt werden. Die Verbindungen I können sowohl als reine Isomere als auch als Isomerengemische zur Anwendung kommen.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide oder fungizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Di-(2-hydroxyeth-1-yl)ammonium, [2-(2-Hydroxy-eth-1-oxy]-eth-1-yl]-ammonium, Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Im Sinne der vorliegenden Erfindung kommen Verbindungen der Formel I mit folgenden Strukturmerkmalen a. - d. bevorzugt in Frage, wobei die Verbindungen eines oder mehrere der Strukturmerkmale a. - d. enthalten können:
a. A unsubstituiertes und substituiertes Phenyl oder Naphthyl.
b. R¹ unsubstituiertes und substituiertes Phenyl.
c. R² Wasserstoff.
d. R³ Wasserstoff; C₁-C₆-Alkyl, insbesondere Methyl, Ethyl, Propyl; C₃-C₇-Cycloalkyl, insbesondere Cyclopropyl.

Für den Fall, daß A oder R¹ Phenyl bedeutet, kann der Phenylring durch einen bis fünf der oben angegebenen Reste substituiert sein, die in 2-, 3- oder 4-Stellung stehen können. Bevorzugt ist der Phenylring ein- oder zweifach substituiert. Als Substituenten kommen bevorzugt in Frage: Halogen, Alkyl, Alkoxy, Nitro, Cyano, Halogenalkyl, Phenyl.

Die Substituenten im Fall von einfach substituierten Phenylringen sind insbesondere die folgenden Gruppen: Halogen, wie z.B. Fluor, Chlor; Alkyl, wie z.B. Methyl; Alkoxy, wie z.B. Methoxy; Nitro; Halogenalkyl, wie z.B. Trifluormethyl; oder Cyano. In diesem Sinne kommen beispielsweise folgende Gruppen in Frage: 4-Halogenphenyl, 4-Alkylphenyl, 4-Alkoxyphenyl, 4-Nitrophenyl, 4-Cyanophenyl, 4-Trifluormethylphenyl, 2-Halogenphenyl, 2-Alkylphenyl, 2-Alkoxyphenyl, 2-Nitrophenyl, 2-Cyanophenyl, 2-Trifluormethylphenyl, 3-Halogenphenyl, 3-Alkylphenyl, 3-Alkoxyphenyl, 3-Nitrophenyl, 3-Cyanophenyl, 3-Trifluormethylphenyl.

Die Substituenten im Fall von zweifach substituierten Phenylringen sind insbesondere die folgenden Gruppen, wobei die Substituenten gleich oder verschieden sein können: Halogen, wie z.B. Chlor; oder Alkyl, wie z.B. Methyl. In diesem Sinne kommen beispielsweise folgende Gruppen in Frage: 2,3-Dihalogenphenyl, 2, 4-Dihalogenphenyl, 2,5-Dihalogenphenyl, 2,6-Dihalogenphenyl, 3, 4-Dihalogenphenyl, 3,5-Dihalogenphenyl, 2,3-Dimethylphenyl, 2,4-Dimethylphenyl, 2,5-Dimethylphenyl, 2,6-Dimethylphenyl, 3,4-Dimethylphenyl, oder 3,5-Dimethylphenyl.

A und R¹ bedeuten vorzugsweise einen unsubstituierten Naphthylrest oder einen unsubstituierten oder substituierten Phenylrest, wobei die Phenylreste durch die zuvor genannten Reste vorzugsweise ein- oder zweifach substituiert sind.

Im Sinne der vorliegenden Erfindung kommen beispielsweise folgende Verbindungen in Tabelle 1 in Frage:

**Tabelle 1**

| | Aryl | R³ | R² | R¹ | Fp. |
|---|---|---|---|---|---|
| 1. | C₆H₅ | H | H | 2-Cl-C₆H₄ | 114 |
| 2. | C₆H₅ | H | H | 3-Cl-C₆H₄ | 123 |
| 3. | C₆H₅ | H | H | 4-Cl-C₆H₄ | 151 |
| 4. | C₆H₅ | H | H | 2,3-Cl₂-C₆H₃ | |
| 5. | C₆H₅ | H | H | 2,4-Cl₂-C₆H₃ | 145 |
| 6. | C₆H₅ | H | H | 2,5-Cl₂-C₆H₃ | |
| 7. | C₆H₅ | H | H | 2,6-Cl₂-C₆H₃ | 184 |
| 8. | C₆H₅ | H | H | 3,4-Cl₂-C₆H₃ | |
| 9. | C₆H₅ | H | H | 3,5-Cl₂-C₆H₃ | 166 |
| 10. | C₆H₅ | H | H | 2-CH₃-C₆H₄ | 138-139 |
| 11. | C₆H₅ | H | H | 3-CH₃-C₆H₄ | 128 |
| 12. | C₆H₅ | H | H | 4-CH₃-C₆H₄ | 134 |
| 13. | C₆H₅ | H | H | 2,3-(CH₃)₂-C₆H₃ | 176 |
| 14. | C₆H₅ | H | H | 2,4-(CH₃)₂-C₆H₃ | 135 |
| 15. | C₆H₅ | H | H | 2,5-(CH₃)₂-C₆H₃ | 164 |
| 16. | C₆H₅ | H | H | 2,6-(CH₃)₂-C₆H₃ | 179 |
| 17. | C₆H₅ | H | H | 3,4-(CH₃)₂-C₆H₃ | |
| 18. | C₆H₅ | H | H | 3,5-(CH₃)₂-C₆H₃ | 136 |
| 19. | C₆H₅ | H | H | 4-CH₃O-C₆H₄ | 127-129 |
| 20. | C₆H₅ | H | H | 4-F-C₆H₄ | 120-127 |
| 21. | C₆H₅ | H | H | 4-CN-C₆H₄ | 114-115 |
| 22. | C₆H₅ | H | H | 4-CF₃-C₆H₄ | 149 |
| 23. | C₆H₅ | H | H | 4-NO₂-C₆H₄ | |
| 24. | C₆H₅ | H | H | 4-C₆H₅-C₆H₄ | |
| 25. | C₆H₅ | H | H | 2-CH₃O-C₆H₄ | |
| 26. | C₆H₅ | H | H | 2-F-C₆H₄ | |
| 27. | C₆H₅ | H | H | 2-CN-C₆H₄ | |
| 28. | C₆H₅ | H | H | 2-CF₃-C₆H₄ | |
| 29. | C₆H₅ | H | H | 2-NO₂-C₆H₄ | |
| 30. | C₆H₅ | H | H | 2-C₆H₅-C₆H₄ | |
| 31. | C₆H₅ | H | H | 3-CH₃O-C₆H₄ | |
| 32. | C₆H₅ | H | H | 3-F-C₆H₄ | |
| 33. | C₆H₅ | H | H | 3-CN-C₆H₄ | |
| 34. | C₆H₅ | H | H | 3-CF₃-C₆H₄ | |
| 35. | C₆H₅ | H | H | 3-NO₂-C₆H₄ | |
| 36. | C₆H₅ | H | H | 3-C₆H₅-C₆H₄ | |
| 37. | C₆H₅ | CH₃ | H | 4-CH₃-C₆H₄ | 105-107 |
| 38. | C₆H₅ | CH₃ | H | 4-Cl-C₆H₄ | 129-131 |
| 39. | C₆H₅ | CH₃ | H | 4-CH₃O-C₆H₄ | 117-118 |
| 40. | C₆H₅ | CH₃ | H | 4-F-C₆H₄ | |
| 41. | C₆H₅ | CH₃ | H | 4-CN-C₆H₄ | |
| 42. | C₆H₅ | CH₃ | H | 4-CF₃-C₆H₄ | |
| 43. | C₆H₅ | CH₃ | H | 4-NO₂-C₆H₄ | |
| 44. | C₆H₅ | CH₃ | H | 4-C₆H₅-C₆H₄ | |
| 45. | C₆H₅ | CH₃ | H | 2-CH₃-C₆H₄ | |
| 46. | C₆H₅ | CH₃ | H | 2-Cl-C₆H₄ | |
| 47. | C₆H₅ | CH₃ | H | 2-CH₃O-C₆H₄ | |
| 48. | C₆H₅ | CH₃ | H | 2-F-C₆H₄ | |
| 49. | C₆H₅ | CH₃ | H | 2-CN-C₆H₄ | |
| 50. | C₆H₅ | CH₃ | H | 2-CF₃-C₆H₄ | |
| 51. | C₆H₅ | CH₃ | H | 2-NO₂-C₆H₄ | |
| 52. | C₆H₅ | CH₃ | H | 2-C₆H₅-C₆H₄ | |
| 53. | C₆H₅ | CH₃ | H | 3-CH₃-C₆H₄ | |
| 54. | C₆H₅ | CH₃ | H | 3-Cl-C₆H₄ | |
| 55. | C₆H₅ | CH₃ | H | 3-CH₃O-C₆H₄ | |
| 56. | C₆H₅ | CH₃ | H | 3-F-C₆H₄ | |
| 57. | C₆H₅ | CH₃ | H | 3-CN-C₆H₄ | |
| 58. | C₆H₅ | CH₃ | H | 3-CF₃-C₆H₄ | |
| 59. | C₆H₅ | CH₃ | H | 3-NO₂-C₆H₄ | |
| 60. | C₆H₅ | CH₃ | H | 3-C₆H₅-C₆H₄ | |
| 61. | C₆H₅ | H | H | C₆H₅ | |
| 62. | C₆H₅ | CH₃ | H | C₆H₅ | |
| 63. | C₆H₅ | C₂H₅ | H | 4-Cl-C₆H₄ | |
| 64. | C₆H₅ | C₃H₇ | H | 4-Cl-C₆H₄ | |
| 65. | C₆H₅ | i.C₃H₇ | H | 4-Cl-C₆H₄ | |
| 66. | C₆H₅ | C₄H₉ | H | 4-Cl-C₆H₄ | |
| 67. | C₆H₅ | c-C₃H₅ | H | 4-Cl-C₆H₄ | |
| 68. | 4-Cl-C₆H₄ | H | H | C₆H₅ | |
| 69. | 4-CH₃-C₆H₄ | H | H | C₆H₅ | |
| 70. | 4-OCH₃ | H | H | C₆H₅ | |
| 71. | 4-NO₂-C₆H₄ | H | H | C₆H₅ | |
| 72. | 4-CN-C₆H₄ | H | H | C₆H₅ | |
| 73. | 4-CF₃-C₆H₄ | H | H | C₆H₅ | |
| 74. | 2-Naphthyl | H | H | C₆H₅ | |
| 75. | 3-Naphthyl | H | H | C₆H₅ | |
| 76. | 4-Cl-C₆H₄ | H | H | 2-Cl-C₆H₄ | |
| 77. | 4-CH₃-C₆H₄ | H | H | 3-Cl-C₆H₄ | |
| 78. | 4-OCH₃ | H | H | 4-Cl-C₆H₄ | |
| 79. | 4-NO₂-C₆H₄ | H | H | 2-CH₃-C₆H₄ | |
| 80. | 4-CN-C₆H₄ | H | H | 3-CH₃-C₆H₄ | |
| 81. | 4-CF₃-C₆H₄ | H | H | 4-CH₃-C₆H₄ | |
| 82. | 2-Naphthyl | H | H | 3,4-(Cl)₂-C₆H₃ | |
| 83. | 3-Naphthyl | H | H | 3,4-(Cl)₂-C₆H₃ | |
| 84. | 4-Cl-C₆H₄ | CH₃ | H | C₆H₅ | |
| 85. | 4-CH₃-C₆H₄ | C₂H₅ | H | C₆H₅ | |
| 86. | 4-OCH₃ | C₃H₇ | H | C₆H₅ | |
| 87. | 4-NO₂-C₆H₄ | C-C₃H₅ | H | C₆H₅ | |
| 88. | 4-CN-C₆H₄ | C₄H₉ | H | C₆H₅ | |
| 89. | 4-CF₃-C₆H₄ | CH₃ | H | C₆H₅ | |
| 90. | 2-Naphthyl | C₂H₅ | H | C₆H₅ | |
| 91. | 3-Naphthyl | c-C₃H₅ | H | C₆H₅ | |
| 92. | 4-Cl-C₆H₄ | H | H | CH2-CH2-(CH₃O)2C₆H₃ | 106 |
| 93. | 3,4-(Cl)₂-C₆H₃ | H | H | C₆H₅ | |
| 94. | 2,4-(Cl)₂-C₆H₃ | H | H | C₆H₅ | |
| 95. | 3-Cl-C₆H₄ | H | H | CH2-CH2-(CH₃O)2C₆H₃ | Öl |
| 96. | 4-CH₃-C₆H₄ | H | H | CH2-CH2-(CH₃O)2C₆H₃ | 113 |
| 97. | C₆H₅ | H | H | CH2-CH2- (CH₃O)2C₆H₃ | 100 |
| 98. | 2-NO₂-C₆H₄ | c-C₃H₅ | H | C₆H₅ | |
| 99. | 4-F-C₆H₄ | H | H | CH2-CH2-(CH₃O)2C₆H₃ | |
| 100. | 4-CF₃-C₆H₄ | H | H | CH2-CH2-(CH₃O)2C₆H₃ | |
| 101. | 2-Naphthyl | C₂H₅ | H | 2-CH₃-C₆H₅ | |
| 102. | 3-Naphthyl | c-C₃H₅ | H | 2-Cl-C₆H₅ | |
| 103. | 3-CH₃-C₆H₄ | H | H | CH2-CH2-(CH₃O)2C₆H₃ | |
| 104. | 3,4-(Cl)₂-C₆H₃ | CH₃ | H | C₆H₅ | |
| 105. | 3-Cl-C₆H₄ | CH₃ | H | C₆H₅ | |
| 106. | 3-CH₃-C₆H₄ | C₂H₅ | H | C₆H₅ | |
| 107. | 3-OCH₃ | C₃H₇ | H | C₆H₅ | |
| 108. | 3-NO₂-C₆H₄ | c-C₃H₅ | H | C₆H₅ | |
| 109. | 3-CN-C₆H₄ | C₄H₉ | H | C₆H₅ | |
| 110. | 3-CF₃-C₆H₄ | CH₃ | H | C₆H₅ | |
| 111. | 4-Cl-C₆H₄ | H | H | 2-Cl-C₆H₄ | 121-122 |
| 112. | 4-Cl-C₆H₄ | H | H | 3-Cl-C₆H₄ | 140-141 |
| 113. | 4-Cl-C₆H₄ | H | H | 4-Cl-C₆H₄ | 162-163 |
| 114. | 4-Cl-C₆H₄ | H | H | 2,3-Cl₂-C₆H₃ | |
| 115. | 4-Cl-C₆H₄ | H | H | 2,4-Cl₂-C₆H₃ | 129-130 |
| 116. | 4-Cl-C₆H₄ | H | H | 2,5-Cl₂-C₆H₃ | |
| 117. | 4-Cl-C₆H₄ | H | H | 2,6-Cl₂-C₆H₃ | 213-215 |
| 118. | 4-Cl-C₆H₄ | H | H | 3,4-Cl₂-C₆H₃ | |
| 119. | 4-Cl-C₆H₄ | H | H | 3,5-Cl₂-C₆H₃ | 201 |
| 120. | 4-Cl-C₆H₄ | H | H | 2-CH₃-C₆H₄ | 147-148 |
| 121. | 4-Cl-C₆H₄ | H | H | 3-CH₃-C₆H₄ | 126-127 |
| 122. | 4-Cl-C₆H₄ | H | H | 4-CH₃-C₆H₄ | 148-149 |
| 123. | 4-Cl-C₆H₄ | H | H | 2,3-(CH₃)₂-C₆H₃ | 162-163 |
| 124. | 4-Cl-C₆H₄ | H | H | 2,4-(CH₃)₂-C₆H₃ | 175 |
| 125. | 4-Cl-C₆H₄ | H | H | 2,5-(CH₃)₂-C₆H₃ | 185 |
| 126. | 4-Cl-C₆H₄ | H | H | 2,6-(CH₃)₂-C₆H₃ | 200-201 |
| 127. | 4-Cl-C₆H₄ | H | H | 3,4-(CH₃)₂-C₆H₃ | |
| 128. | 4-Cl-C₆H₄ | H | H | 3,5-(CH₃)₂-C₆H₃ | 190-191 |
| 129. | 4-Cl-C₆H₄ | H | H | 4-CH₃O-C₆H₄ | 127-130 |
| 130. | 4-Cl-C₆H₄ | H | H | 4-F-C₆H₄ | |
| 131. | 4-Cl-C₆H₄ | H | H | 4-CN-C₆H₄ | 110 |
| 132. | 4-Cl-C₆H₄ | H | H | 4-CF₃-C₆H₄ | 168 |
| 133. | 4-CH₃-C₆H₄ | H | H | 2-Cl-C₆H₄ | 115 |
| 134. | 4-CH₃-C₆H₄ | H | H | 3-Cl-C₆H₄ | 137 |
| 135. | 4-CH₃-C₆H₄ | H | H | 4-Cl-C₆H₄ | 113 |
| 136. | 4-CH₃-C₆H₄ | H | H | 2,3-Cl₂-C₆H₃ | |
| 137. | 4-CH₃-C₆H₄ | H | H | 2,4-Cl₂-C₆H₃ | 146 |
| 138. | 4-CH₃-C₆H₄ | H | H | 2,5-Cl₂-C₆H₃ | |
| 139. | 4-CH₃-C₆H₄ | H | H | 2,6-Cl₂-C₆H₃ | |
| 140. | 4-CH₃-C₆H₄ | H | H | 3,4-Cl₂-C₆H₃ | |
| 141. | 4-CH₃-C₆H₄ | H | H | 3,5-Cl₂-C₆H₃ | 199-201 |
| 142. | 4-CH₃-C₆H₄ | H | H | 2-CH₃-C₆H₄ | 158-159 |
| 143. | 4-CH₃-C₆H₄ | H | H | 3-CH₃-C₆H₄ | 116-117 |
| 144. | 4-CH₃-C₆H₄ | H | H | 4-CH₃-C₆H₄ | |
| 145. | 4-CH₃-C₆H₄ | H | H | 2,3-(CH₃)₂-C₆H₃ | |
| 146. | 4-CH₃-C₆H₄ | H | H | 2,4-(CH₃)₂-C₆H₃ | |
| 147. | 4-CH₃-C₆H₄ | H | H | 2,5-(CH₃)₂-C₆H₃ | 148-149 |
| 148. | 4-CH₃-C₆H₄ | H | H | 2,6-(CH₃)₂-C₆H₃ | 181-182 |
| 149. | 4-CH₃-C₆H₄ | H | H | 3,4-(CH₃)₂-C₆H₃ | |
| 150. | 4-CH₃-C₆H₄ | H | H | 3,5-(CH₃)₂-C₆H₃ | 157-158 |
| 151. | 4-CH₃-C₆H₄ | H | H | 4-CH₃O-C₆H₄ | |
| 152. | 4-CH₃-C₆H₄ | H | H | 4-F-C₆H₄ | 166-167 |
| 153. | 4-CH₃-C₆H₄ | H | H | 4-CN-C₆H₄ | |
| 154. | 4-CH₃-C₆H₄ | H | H | 4-CF₃-C₆H₄ | |
| 155. | C₆H₅ | CH₃ | H | 2,4-Cl₂-C₆H₃ | Öl |
| 156. | C₆H₅ | CH₃ | H | 2,4-(CH₃)₂-C₆H₃ | 82-83 |
| 157. | C₆H₅ | CH₃ | H | 2,4-(CH₃O)₂-C₆H₃ | Öl |
| 158. | C₆H₅ | CH₃ | H | | |
| 159. | C₆H₅ | CH₃ | H | | |
| 160. | C₆H₅ | i-C₃H₇ | H | 2,4-(CH₃O)₂-C₆H₃ | Öl |
| 161. | C₆H₅ | i-C₃H₇ | H | 2-CH₃-C₆H₄ | 102 |
| 162. | C₆H₅ | i-C₃H₇ | H | 4-CH₃-C₆H₄ | Öl |
| 163. | C₆H₅ | i-C₃H₇ | H | 2,4-CH₃-C₆H₃ | 118-119 |
| 164. | C₆H₅ | i-C₃H₇ | H | 2-CH₃O-C₆H₄ | Öl |
| 165. | C₆H₅ | i-C₃H₇ | H | 4-CH₃O-C₆H₄ | Öl |
| 166. | C₆H₅ | i-C₃H₇ | H | 2-CN-C₆H₄ | Öl |
| 167. | C₆H₅ | i-C₃H₇ | H | 4-CN-C₆H₄ | Öl |
| 168. | C₆H₅ | C₂H₅ | H | 2-Cl-C₆H₄ | Öl |
| 169. | C₆H₅ | C₂H₅ | H | 4-Cl-C₆H₄ | 106 |
| 170. | C₆H₅ | C₂H₅ | H | 2-CH₃-C₆H₄ | Öl |
| 171. | C₆H₅ | C₂H₅ | H | 4-CH₃-C₆H₄ | 93-94 |
| 172. | 4-CH₃-C₆H₄ | CH₃ | H | 2-Cl-C₆H₄ | Öl |
| 173. | C₆H₅ | C₂H₅ | H | 2,4-Cl₂-C₆H₃ | Öl |
| 174. | C₆H₅ | C₂H₅ | H | 2,4-(CH₃)₂-C₆H₃ | Öl |
| 175. | C₆H₅ | C₂H₅ | H | 2-CH₃O-C₆H₄ | Öl |
| 176. | C₆H₅ | C₂H₅ | H | 4-CH₃O-C₆H₄ | Öl |
| 177. | C₆H₅ | C₂H₅ | H | 2,4-(CH₃O)₂-C₆H₃ | Öl |
| 178. | C₆H₅ | C₂H₅ | H | 2-CN-C₆H₄ | Öl |
| 179. | C₆H₅ | C₂H₅ | H | 4-CN-C₆H₄ | Öl |
| 180. | 4-CH₃-C₆H₄ | CH₃ | H | 4-Cl-C₆H₄ | 82 |
| 181. | 4-CH₃-C₆H₄ | CH₃ | H | 2-CH₃-C₆H₄ | 75-76 |
| 182. | 4-CH₃-C₆H₄ | CH₃ | H | 4-CH₃-C₆H₄ | Öl |
| 183. | 4-CH₃-C₆H₄ | CH₃ | H | 2,4-Cl₂-C₆H₃ | 122-124 |
| 184. | 4-CH₃-C₆H₄ | CH₃ | H | 2,4-(CH₃)₂-C₆H₃ | 120-121 |
| 185. | 4-CH₃-C₆H₄ | CH₃ | H | 2-CH₃O-C₆H₄ | Öl |
| 186. | 4-CH₃-C₆H₄ | CH₃ | H | 4-CH₃O-C₆H₄ | Öl |
| 187. | 4-CH₃-C₆H₄ | CH₃ | H | 2,4-(CH₃O)₂-C₆H₃ | Öl |
| 188. | 4-CH₃-C₆H₄ | CH₃ | H | 2-CN-C₆H₄ | 130-132 |
| 189. | 4-CH₃-C₆H₄ | CH₃ | H | 4-CN-C₆H₄ | Öl |
| 190. | C₆H₅ | i-C₃H₇ | H | 2-Cl-C₆H₄ | Öl |
| 191. | 4-Cl-C₆H₄ | H | H | 2-CN-C₆H₄ | 141-142 |
| 192. | 4-Cl-C₆H₄ | H | H | 2-OCH₃-C₆H₄ | 139 |
| 193. | 4-Cl-C₆H₄ | H | H | 2,4-(CH₃O)₂-C₆H₃ | 177 |
| 194. | 4-CN-C₆H₄ | H | H | 2,4-(CH₃)₂-C₆H₃ | 168-169 |
| 195. | 4-CN-C₆H₄ | H | H | 2,4-(Cl)₂-C₆H₃ | 189 |
| 196. | 4-CN-C₆H₄ | H | H | 4-CN-C₆H₃ | 187 |
| 197. | 4-CN-C₆H₄ | H | H | 4-Cl-C₆H₃ | 204 |
| 198. | 4-CN-C₆H₄ | H | H | 4-CH₃-C₆H₃ | 149 |
| 199. | 4-CN-C₆H₄ | H | H | 4-OCH₃-C₆H₃ | 149 |
| 200. | 4-CN-C₆H₄ | H | H | 2,4-(CH₃O)₂-C₆H₃ | 165 |
| 201. | 4-CN-C₆H₄ | H | H | 2-Cl-C₆H₄ | 158 |
| 202. | 4-CN-C₆H₄ | H | H | 2-CH₃-C₆H₄ | 164 |
| 203. | 4-CN-C₆H₄ | H | H | 2-OCH₃-C₆H₄ | 60-66 |
| 204. | 4-CH₃O-C₆H₄ | H | H | 2-CH₃-C₆H₄ | 171-176 |
| 205. | 4-CH₃O-C₆H₄ | H | H | 4-Cl-C₆H₄ | 151-155 |
| 206. | 4-CH₃O-C₆H₄ | H | H | 4-CH₃-C₆H₄ | 130-132 |
| 207. | 4-CH₃O-C₆H₄ | H | H | 2,4-(CH₃)₂-C₆H₃ | 138-140 |
| 208. | 4-CH₃-C₆H₄ | i-C₃H₇ | H | 4-tBu-C₆H₄ | 134-136 |
| 209. | 4-CH₃-C₆H₄ | i-C₃H₇ | H | 4-i-C₃H₇-C₆H₄ | 102-104 |
| 210. | 4-CH₃-C₆H₄ | i-C₃H₇ | H | 4-F-C₆H₄ | Öl |
| 211. | 4-CH₃-C₆H₄ | i-C₃H₇ | H | 2-F-C₆H₄ | 80-81 |
| 212. | 4-CH₃-C₆H₄ | i-C₃H₇ | H | 4-CN-C₆H₄ | 83 |
| 213. | 4-CH₃-C₆H₄ | i-C₃H₇ | H | 2-CN-C₆H₄ | Öl |
| 214. | 4-CH₃-C₆H₄ | i-C₃H₇ | H | 2,4-(CH₃O)₂-C₆H₃ | Öl |
| 215. | 4-CH₃-C₆H₄ | i-C₃H₇ | H | 4-OCH₃-C₆H₄ | Öl |
| 216. | 4-CH₃-C₆H₄ | i-C₃H₇ | H | 2-OCH₃-C₆H₄ | 84-85 |
| 217. | 4-CH₃-C₆H₄ | i-C₃H₇ | H | 2, 4-(CH₃)₂-C₆H₃ | 99 |
| 218. | 4-CH₃-C₆H₄ | i-C₃H₇ | H | 2,4-(Cl)₂-C₆H₃ | Öl |
| 219. | 4-CH₃-C₆H₄ | i-C₃H₇ | H | 4-CH₃-C₆H₄ | 96-97 |
| 220. | 4-CH₃-C₆H₄ | i-C₃H₇ | H | 2-CH₃-C₆H₄ | 133-135 |
| 221. | 4-CH₃-C₆H₄ | i-C₃H₇ | H | 4-Cl-C₆H₄ | 98-99 |
| 222. | 4-CH₃-C₆H₄ | i-C₃H₇ | H | 2-Cl-C₆H₄ | Öl |
| 223. | 3-Cl-C₆H₄ | H | H | 2-Cl-C₆H₄ | 113-114 |
| 224. | 3-Cl-C₆H₄ | H | H | 4-Cl-C₆H₄ | 122-124 |
| 225. | 3-Cl-C₆H₄ | H | H | 2-CH₃-C₆H₄ | 142-143 |
| 226. | 3-Cl-C₆H₄ | H | H | 4-CH₃-C₆H₄ | 114 |
| 227. | 3-Cl-C₆H₄ | H | H | 2,4-(Cl)₂-C₆H₃ | 143 |
| 228. | 3-Cl-C₆H₄ | H | H | 2,4-(CH₃)₂-C₆H₃ | 118-119 |
| 229. | 3-Cl-C₆H₄ | H | H | 2-CH₃O-C₆H₄ | 102 |
| 230. | 3-Cl-C₆H₄ | H | H | 4-CH₃O-C₆H₄ | 138 |
| 231. | 3-Cl-C₆H₄ | H | H | 2,4-(CH₃O)₂-C₆H₄ | 118-119 |
| 232. | 3-Cl-C₆H₄ | H | H | 4-CN-C₆H₄ | 47-55 |
| 233. | 3-Cl-C₆H₄ | H | H | 2-F-C₆H₄ | 128-131 |
| 234. | 3-Cl-C₆H₄ | H | H | 4-F-C₆H₄ | 132-133 |
| 235. | 3-Cl-C₆H₄ | H | H | 4-i-C₃H₇-C₆H₄ | 131-133 |
| 236. | 3-Cl-C₆H₄ | H | H | 4-t-C₄H₉-C₆H₄ | 123-125 |
| 237. | 4-CH₃O-C₆H₄ | H | H | 2,4-(CL)₂-C₆H₃ | 132 |
| 238. | 4-CH₃O-C₆H₄ | H | H | 2,4-(CH₃O)₂-C₆H₃ | 115-118 |
| 239. | 4-CH₃O-C₆H₄ | H | H | 2-CH₃O-C₆H₄ | 130 |
| 240. | 4-CH₃O-C₆H₄ | H | H | 2-CN-C₆H₄ | 138-139 |
| 241. | 4-CH₃O-C₆H₄ | H | H | 4-CH₃O-C₆H₄ | 142-143 |
| 242. | 4-CH₃O-C₆H₄ | H | H | 4-CN-C₆H₄ | 128-129 |
| 243. | 4-CH₃O-C₆H₄ | H | H | 2-F-C₆H₄ | 132 |
| 244. | 4-CH₃O-C₆H₄ | H | H | 2-Cl-C₆H₄ | 120-121 |
| 245. | 4-Cl-C₆H₄ | H | H | 2-F-C₆H₄ | 126-127 |
| 246. | 4-CN-C₆H₄ | H | H | 2-F-C₆H₄ | 146-147 |
| 247. | 4-Cl-C₆H₄ | H | H | 4-i-C₃H₇-C₆H₄ | 148-149 |
| 248. | 4-Cl-C₆H₄ | H | H | 4-t-C₄H₉-C₆H₄ | 161-162 |
| 249. | 4-CN-C₆H₄ | H | H | 4-F-C₆H₄ | 183-185 |
| 250. | 4-CN-C₆H₄ | H | H | 4-i-C₃H₇-C₆H₄ | 140-149 |
| 251. | 4-CN-C₆H₄ | H | H | 4-t-Bu-C₆H₄ | 89-93 |
| 252. | 4-CH₃O-C₆H₄ | H | H | 4-F-C₆H₄ | 127-128 |
| 253. | 4-CH₃O-C₆H₄ | H | H | 4-i-C₃H₇-C₆H₄ | 106-110 |
| 254. | 4-CH₃-C₆H₄ | C₂H₅ | H | 2-Cl-C₆H₄ | Öl |
| 255. | 4-CH₃-C₆H₄ | C₂H₅ | H | 2,4-(Cl)₂-C₆H₃ | Öl |
| 256. | 4-CH₃-C₆H₄ | C₂H₅ | H | 2,4-(CH₃)₂-C₆H₃ | 90-92 |
| 257. | 4-CH₃-C₆H₄ | C₂H₅ | H | 2,4-(CH₃O)₂-C₆H₃ | Öl |
| 258. | 4-CH₃-C₆H₄ | C₂H₅ | H | 4-Cl-C₆H₄ | 95-96 |
| 259. | 4-CH₃-C₆H₄ | C₂H₅ | H | 2-CH₃-C₆H₄ | 89-90 |
| 260. | 4-CH₃-C₆H₄ | C₂H₅ | H | 4-CH₃-C₆H₄ | 92-94 |
| 261. | 4-CH₃-C₆H₄ | C₂H₅ | H | 2-CH₃O-C₆H₄ | Öl |
| 262. | 4-CH₃-C₆H₄ | C₂H₅ | H | 4-CH₃O-C₆H₄ | Öl |
| 263. | 4-CH₃-C₆H₄ | C₂H₅ | H | 2-CN-C₆H₄ | 133-135 |
| 264. | 4-CH₃-C₆H₄ | C₂H₅ | H | 4-CN-C₆H₄ | 135-136 |
| 265. | 4-CH₃-C₆H₄ | C₂H₅ | H | 2-F-C₆H₄ | Öl |
| 266. | 4-CH₃-C₆H₄ | C₂H₅ | H | 4-F-C₆H₄ | Öl |
| 267. | 4-CH₃-C₆H₄ | C₂H₅ | H | 4-i-C₃H₇-C₆H₄ | Öl |
| 268. | 4-CH₃-C₆H₄ | C₂H₅ | H | 4-t-Bu-C₆H₄ | 105 |
| 269. | 4-CH₃Ol-C₆H₄ | C₂H₅ | H | 4-t-Bu-C₆H₄ | 65-67 |
| 270. | 4-Cl-C₆H₄ | C₂H₅ | H | 2-CH₃-C₆H₄ | 96-97 |
| 271. | 4-Cl-C₆H₄ | C₂H₅ | H | 4-Cl-C₆H₄ | 128 |
| 272. | 4-Cl-C₆H₄ | C₂H₅ | H | 4-CH₃-C₆H₄ | 87-88 |
| 273. | 4-Cl-C₆H₄ | C₂H₅ | H | 2,4-(Cl)₂-C₆H₃ | Öl |
| 274. | 4-Cl-C₆H₄ | C₂H₅ | H | 2,4-(CH₃)₂-C₆H₃ | 112 |
| 275. | 4-Cl-C₆H₄ | C₂H₅ | H | 2-CH₃O-C₆H₄ | Öl |
| 276. | 4-Cl-C₆H₄ | C₂H₅ | H | 4-CH₃O-C₆H₄ | 90-92 |
| 277. | 4-Cl-C₆H₄ | C₂H₅ | H | 2,4-(CH₃O)₂-C₆H₃ | Öl |
| 278. | 4-Cl-C₆H₄ | C₂H₅ | H | 4-CN-C₆H₄ | 154 |
| 279. | 3-CH₃-C₆H₄ | H | H | 2-Cl-C₆H₄ | 71 |
| 280. | 3-CH₃-C₆H₄ | H | H | 4-Cl-C₆H₄ | 117 |
| 281. | 3-CH₃-C₆H₄ | H | H | 2-CH₃-C₆H₄ | 107-109 |
| 282. | 3-CH₃-C₆H₄ | H | H | 4-CH₃-C₆H₄ | 112-113 |
| 283. | 3-CH₃-C₆H₄ | H | H | 2,4-(Cl)₂-C₆H₃ | 100-102 |
| 284. | 3-CH₃-C₆H₄ | H | H | 2,4-(CH₃)₂-C₆H₃ | 124-126 |
| 285. | 3-CH₃-C₆H₄ | H | H | 2,4-(CH₃O)₂-C₆H₃ | 102 |
| 286. | 3-CH₃-C₆H₄ | H | H | 2-CH₃O-C₆H₄ | 124-125 |
| 287. | 3-CH₃-C₆H₄ | H | H | 4-CH₃O-C₆H₄ | 105-107 |
| 288. | 3-CH₃-C₆H₄ | H | H | 2-CN-C₆H₄ | 94-98 |
| 289. | 3-CH₃-C₆H₄ | H | H | 4-CN-C₆H₄ | 89-90 |
| 290. | 3-CH₃-C₆H₄ | H | H | 2-F-C₆H₄ | 100-101 |
| 291. | 3-CH₃-C₆H₄ | H | H | 4-F-C₆H₄ | 91-93 |
| 292. | 3-CH₃-C₆H₄ | H | H | 4-C₃H₇-C₆H₄ | 93-94 |
| 293. | 3-CH₃-C₆H₄ | H | H | 4-t-C₄H₉-C₆H₄ | 77-79 |
| 294. | 3-CH₃-C₆H₄ | H | H | 3,4-(Cl)₂-C₆H₃ | 134-136 |
| 295. | 3-CH₃-C₆H₄ | H | H | 3,4-(CH₃)₂-C₆H₃ | 123-125 |
| 296. | 3-CH₃-C₆H₄ | H | H | 3,4-(F)₂-C₆H₃ | 109-110 |
| 297. | 3-CH₃-C₆H₄ | H | H | 3,4-(CH₃O)₂-C₆H₃ | Öl |
| 298. | 3-Cl-C₆H₄ | H | H | 3,4-(F)₂-C₆H₃ | 133-134 |
| 299. | 3-Cl-C₆H₄ | H | H | 3,4-(CH₃O)₂-C₆H₃ | 192-193 |
| 300. | 3-Cl-C₆H₄ | H | H | 3,4-(Cl)₂-C₆H₃ | 177 |
| 301. | 3-Cl-C₆H₄ | H | H | 3,4-(CH₃)₂-C₆H₃ | 97 |
| 302. | 4-Cl-C₆H₄ | H | H | 3,4-(Cl)₂-C₆H₃ | 213-214 |
| 303. | 4-Cl-C₆H₄ | H | H | 3,4-(CH₃)₂-C₆H₃ | 153-154 |
| 304. | 4-Cl-C₆H₄ | H | H | 3,4-(F)₂-C₆H₃ | 150-151 |
| 305. | 4-Cl-C₆H₄ | H | H | 3,4-(CH₃O)₂-C₆H₃ | 167 |
| 306. | 4-CN-C₆H₄ | H | H | 3,4-(F)₂-C₆H₃ | 178-179 |
| 307. | 4-CN-C₆H₄ | H | H | 3,4-(CH₃)₂-C₆H₃ | 144 |
| 308. | 4-CN-C₆H₄ | H | H | 3,4-(CH₃O)₂-C₆H₃ | |
| 309. | 4-CN-C₆H₄ | H | H | 3,4-(Cl)₂-C₆H₃ | 260 |
| 310. | 4-CH₃O-C₆H₄ | H | H | 3,4-(Cl)₂-C₆H₃ | 162-163 |
| 311. | 4-CH₃O-C₆H₄ | H | H | 3,4-(CH₃)₂-C₆H₃ | 122-123 |
| 312. | 4-CH₃O-C₆H₄ | H | H | 3,4-(F)₂-C₆H₃ | 148 |
| 313. | 4-Cl-C₆H₄ | C₂H₅ | H | 2-Cl-C₆H₄ | Öl |
| 314. | 4-Cl-C₆H₄ | i-C₃H₇ | H | 2,4-(Cl)₂-C₆H₃ | 113 |
| 315. | 4-Cl-C₆H₄ | i-C₃H₇ | H | 2,4-(CH₃O)₂-C₆H₃ | Öl |
| 316. | 4-Cl-C₆H₄ | i-C₃H₇ | H | 2,4-(CH₃)₂-C₆H₃ | 118 |
| 317. | 4-Cl-C₆H₄ | i-C₃H₇ | H | 2-Cl-C₆H₄ | Öl |
| 318. | 4-Cl-C₆H₄ | i-C₃H₇ | H | 3-Cl-C₆H₄ | 110-111 |
| 319. | 4-Cl-C₆H₄ | i-C₃H₇ | H | 4-Cl-C₆H₄ | 126 |
| 320. | 4-Cl-C₆H₄ | i-C₃H₇ | H | 2-CH₃-C₆H₄ | 138 |
| 321. | 4-Cl-C₆H₄ | i-C₃H₇ | H | 4-CH₃-C₆H₄ | 95-96 |
| 322. | 4-Cl-C₆H₄ | i-C₃H₇ | H | 4-CN-C₆H₄ | 167-168 |
| 323. | 4-Cl-C₆H₄ | i-C₃H₇ | H | 2-CN-C₆H₄ | 109 |
| 324. | 4-Cl-C₆H₄ | i-C₃H₇ | H | 4-CH₃O-C₆H₄ | Öl |
| 325. | 3-CF₃-C₆H₄ | H | H | 2-Cl-C₆H₄ | 93-95 |
| 326. | 3-CF₃-C₆H₄ | H | H | 4-Cl-C₆H₄ | 166-167 |
| 327. | 3-CF₃-C₆H₄ | H | H | 2-CH₃-C₆H₄ | 141-142 |
| 328. | 3-CF₃-C₆H₄ | H | H | 4-CH₃-C₆H₄ | 144-146 |
| 329. | 3-CF₃-C₆H₄ | H | H | 2-CH₃O-C₆H₄ | Öl |
| 330. | 3-CF₃-C₆H₄ | H | H | 4-CH₃O-6H₄ | 138 |
| 331. | 3-CF₃-C₆H₄ | H | H | 4-CN-C₆H₄ | 122 |
| 332. | 3-CF₃-C₆H₄ | H | H | 2-F-C₆H₄ | 85-86 |
| 333. | 3-CF₃-C₆H₄ | H | H | 4-F-C₆H₄ | 133-134 |
| 334. | 3-CF₃-C₆H₄ | H | H | 4-i-C₃H₇-C₆H₄ | 127-128 |
| 335. | 3-CF₃-C₆H₄ | H | H | 4-t-C₄H₉-C₆H₄ | 133-134 |
| 336. | 3-CF₃-C₆H₄ | H | H | 3,4-(Cl)₂-C₆H₃ | 178-179 |
| 337. | 3-CF₃-C₆H₄ | H | H | 3,4-(CH₃)₂-C₆H₃ | 116 |
| 338. | 3-CF₃-C₆H₄ | H | H | 3,4-(F)₂-C₆H₃ | 110 |
| 339. | 3-CF₃-C₆H₄ | H | H | 2,4-(CH₃)₂-C₆H₃ | 95-96 |
| 340. | 3-CF₃-C₆H₄ | H | H | 2,4-(Cl)₂-C₆H₃ | 120 |
| 341. | 3-CF₃-C₆H₄ | H | H | 3,4-(CH₃O)₂-C₆H₃ | 144 |
| 342. | 3-CF₃-C₆H₄ | H | H | 2,4-(CH₃O)₂-C₆H₃ | 125 |
| 343. | 3,5-(Cl)₂C₆H₃ | H | H | 2-Cl-C₆H₄ | 160 |
| 344. | 3,5-(Cl)₂C₆H₃ | H | H | 4-Cl-C₆H₄ | 214 |
| 345. | 3,5-(Cl)₂C₆H₃ | H | H | 2-CH₃-C₆H₄ | 180-182 |
| 346. | 3,5-(Cl)₂C₆H₃ | H | H | 4-CH₃-C₆H₄ | 166 |
| 347. | 3,5-(Cl)₂C₆H₃ | H | H | 2-CH₃O-C₆H₄ | 164 |
| 348. | 3,5-(Cl)₂C₆H₃ | H | H | 4-CH₃O-C₆H₄ | 181 |
| 349. | 3,5-(Cl)₂C₆H₃ | H | H | 2-F-C₆H₄ | 169 |
| 350. | 3,5-(Cl)₂C₆H₃ | H | H | 4-CN-C₆H₄ | 160 |
| 351. | 3,5-(Cl)2C₆H₃ | H | H | 4-F-C₆H₄ | 164 |
| 352. | 3,5-(Cl)₂C₆H₃ | H | H | 4-i-C₃H₇-C₆H₄ | Öl |
| 353. | 3,5-(Cl)₂C₆H₃ | H | H | 4-t-C₄H₉-C₆H₄ | 158-160 |
| 354. | 3,5-(Cl)₂C₆H₃ | H | H | 2,4-(Cl)₂-C₆H₃ | 153-155 |
| 355. | 3,5-(Cl)₂C₆H₃ | H | H | 2,4-(CH₃)₂-C₆H₃ | 205 |
| 356. | 3,5-(Cl)₂C₆H₃ | H | H | 2,4-(CH₃O)₂-C₆H₃ | 175 |
| 357. | 3,5-(Cl)₂C₆H₃ | H | H | 3,4-(Cl)₂-C₆H₃ | 226-229 |
| 358. | 3,5-(Cl)₂C₆H₃ | H | H | 3,4-(CH₃)₂-C₆H₃ | 145 |
| 359. | 3,5-(Cl)₂C₆H₃ | H | H | 3,4-(F)₂-C₆H₃ | Öl |
| 360. | 3,5-(Cl)₂C₆H₃ | H | H | 3,4-(CH₃O)₂-C₆H₃ | Öl |

Die Verbindungen der Formel I können analog zu den in der Literatur beschriebenen Verfahren (vgl. DD 156663, J.Am.chem.Soc. 39, 1908, 63) hergestellt werden. Beispielsweise erfolgt die Synthese nach dem folgenden Standardverfahren, wobei die Phenylringe in Formel II oder III gegebenenfalls auch substituiert sein können: 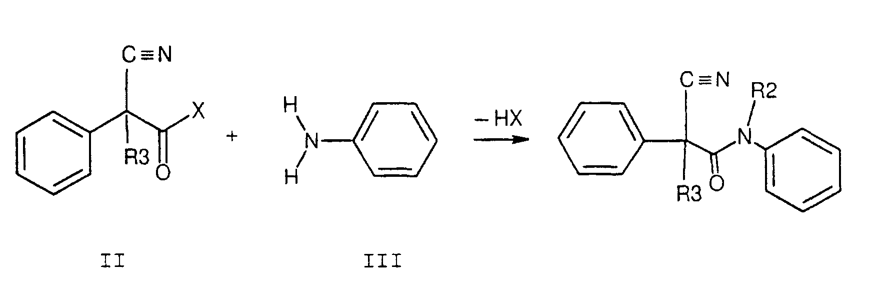 X bedeutet eine geeignete Abgangsgruppe, wie z.B. Halogen oder Alkoxy.

Die Cyanocarbonsäurederivate der Formel II können nach literaturbekannten Verfahren hergestellt werden (vgl. Org. Synthesis, Collect Vol IV, 1963, S. 461 ff.). Die Phenylamine der Formel III sind entweder kommerziell erhältlich bzw. können ebenfalls nach literaturbekannten Verfahren hergestellt werden.

Ein weiterer Gegenstand der vorliegenden Erfindung ist ein Verfahren zur Behandlung von Pflanzen zur Bekämpfung von Schadpilzen; zur Bekämpfung von Schadpilzen; oder zur Bekämpfung unerwünschten Wuchses von Schadpflanzen, dadurch gekennzeichnet, daß man
i) die Pflanzen oder deren Lebensraum,
ii) die Pilze, deren Lebensraum oder die vor Pilzbefall zu schützenden Materialien, Pflanzen, Samen, Böden, Flächen oder Räume oder
iii) die Schadpflanzen oder deren Lebensraum
mit einer wirksamen Menge einer Verbindung der in Anspruch 1 angegebenen Formel I, worin die Reste die in einem der Ansprüche 1 bis 11 angegebene Bedeutung haben, oder einem diese Verbindung enthaltenden Mittel in Kontakt bringt, mit Ausnahme der Verbindungen, in denen A eine unsubstituierte Phenylgruppe; R³ Wasserstoff oder C₁- bis C₅-Alkyl bedeutet; und R¹ Aryl oder Aryl, das durch Halogen substituiert ist, oder Arylalkyl ist.

Die Verbindungen der Formel I eignen sich als Wirkstoffe zur Herstellung von Pflanzenschutzmitteln. Als Pflanzenschutzmittel im Sinne der vorliegenden Erfindung versteht man in der Regel Mischungen von Wirkstoffen der Formel I mit Zusatz- oder Hilfsstoffen, die die Anwendung der Mischungen in der Landwirtschaft oder im Gartenbau und die Ausbringung dieser Mittel auf Freiflächen oder zur Behandlung von Pflanzen ermöglichen. Vorzugsweise können die Pflanzenschutzmittel als Herbizide und/oder Fungizide verwendet werden.

Normalerweise werden die Pflanzen mit den Wirkstoffen oder den Pflanzenschutzmittelpräparaten besprüht oder bestäubt oder die Samen der Pflanzen mit den Wirkstoffen behandelt.

Die Verbindungen der Formel I und deren landwirtschaftlich brauchbaren Salze eignen sich - sowohl als Stereoisomerengemische als auch in Form der reinen Stereoisomeren - vor allem als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlichen brauchbaren Salzes von I und für die Formulierung von Pflanzenschutzmitteln üblichen Hilfsmittel.

Als inerte Zusatzstoffe kommen im wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen der Formel I als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
a) 20 Gewichtsteile einer Verbindung I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
b) 20 Gewichtsteile einer Verbindung I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
c) 20 Gewichtsteile einer Verbindung I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
d) 20 Gewichtsteile einer Verbindung I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
e) 3 Gewichtsteile einer Verbindung I werden mit 97 Gewichtsteilen feinteiligem Kaolin vermischt. Man erhält auf diese Weise ein Stäubemittel, das 3 Gew.-% des Wirkstoffs enthält.
f) 20 Gewichtsteile einer Verbindung I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
g) 1 Gewichtsteil einer Verbindung I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
h) 1 Gewichtsteil einer Verbindung I wird in einer Mischung gelöst, die aus 80 Gewichtsteilen Cyclohexanon und 20 Gewichtsteilen Wettol [ EM 31 (nicht ionischer Emulgator auf der Basis von ethoxyliertem Ricinusöl) besteht. Man erhält ein stabiles Emulsionskonzentrat.

Die Applikation der Wirkstoffe der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy/Hetaryloxyalkansäure und deren Derivate, Benzoesäure und deren Derivate, Benzothiadiazinone, 2-(Aroyl/Hetaroyl)-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.)

Verbindungen der Formel I besitzen ferner eine fungizide Wirkung. Sie zeichnen sich insbesondere durch eine hervorragende Wirkung gegen ein breites Spektrum von pflanzenpathogenen Pilzen aus, vor allem aus der Klasse der Ascomyceten, Deuteromyceten, Phycomyceten und Basidiomyceten. Sie sind z.T. systemisch wirksam und können daher auch als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Wirkstoffe der Formel I können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken: Fungizide aus der Wirkstoffgruppe der Schwefel-, Dithiocarbamate und deren Derivate; Nitroderivate; heterocyclische Substanzen, wie z.B. Imidazolinderivate, Triazole, Triazinderivate, Chinoxalinderivate, Benzimidazolderivate, Thiadiazolderivate, etc.; oder Strobilurine.

Die Erfindung wird anhand der folgenden Ausführungsbeispiele näher erläutert:

### Beispiel 1

### 2-Cyano-2-phenylessigsäure-N-(3-chlorphenyl)amid (Verbindung Nr. 2 aus Tabelle 1)

3 g (0,016 mol) Phenylcyanessigsäureethylester und 2 g (0,016 mol) 3-Chloranilin werden in Substanz 5 Std. auf 130 C erhitzt. Anschließend wird in 100 ml Dichlormethan aufgenommen und 3mal mit 10%-iger Salzsäure extrahiert. Die organische Phase wird abgetrennt, getrocknet und eingeengt. Der erhaltene Rückstand wird in 30 ml Dichlormethan aufgenommen und 200 ml Petrolether zugegeben. Der erhaltene kristalline Niederschlag wird abgesaugt, mit Petrolether nachgewaschen und getrocknet. Ausbeute 38%; Fp. 123°C.

### Beispiel 2

### Herbizide Wirkung

Die herbizide Wirkung der erfindungsgemäßen Verbindungen wird im Rahmen des folgenden repräsentativen Testmodells untersucht. Der Einfluß der Testsubstanzen auf das Wachstum der Wasserlinse wird nach dem folgenden Verfahren bestimmt:

Die Anzucht der Wasserlinse *Lemna paucicostata* erfolgte unter sterilen Bedinungen in 250 ml Glasgefäßen mit 100 ml anorganischer Nährlösung und Zugabe von 1 % Saccharose, wie bei Grossmann et al., Pesticide Science 35, 283-289 (1992) beschrieben. Zu Beginn des Tests wurden 150 µl einer acetonischen Wirkstofflösung (Stammlösung: 100fach konzentriert) in Petrischalen (Durchmesser 6 cm, Höhe 1,5 cm; Greiner, Frickenhausen) mit 15 ml Nährlösung (ohne Saccharosezusatz) pipettiert. Den Kontrolltests wurde nur der Lösungsmittelanteil der Wirkstofflösung zur Nährlösung zugegeben. Anschließend wurden 4 *Lemna*-Pflanzen pro Schale eingesetzt, die Schalen mit Deckeln abgedeckt und unter Dauerlicht-Bedingungen bei 25°C inkubiert. Nach 8 - 10 Tagen wurde mit einem Bildanalysegerät (Fa. Imago, Compulog Computer Syst., Böblingen) die Zunahme der Blattfläche als Wachstumsparameter bestimmt und daraus die Wachstumshemmung in % zur Kontrolle berechnet.

Die Ergebnisse der Tests sind der folgenden Tabelle zu entnehmen. Die herbizide Wirkung der erfinungsgemäßen Verbindungen zeigt sich in der starken Hemmung des Wachstums von *Lemna*.

| Verbindung | Konzentration (µmolar) | Wachstumshemmung (% zur Kontrolle) |
|---|---|---|
| A | 100 | 100 |
| | 10 | 93 |
| | 1 | 40 |
| A = 2-Cyano-2-phenylessigsäure-(N-3,4-dichlorphenyl)amid (Tabelle 1, Verb. Nr. 8) | | |

### Beispiel 3

### Fungizide Wirkung

### Wirksamkeit gegen Pyricularia oryzae (protektiv)

Blätter von in Töpfen gewachsenen Reiskeimlingen der Sorte "Tai-Nong 67" wurden mit wäßriger Wirkstoffaufbereitung, die mit einer Stammlösung aus 10 % Wirkstoff, 63 % Cyclohexanon und 27 % Emulgiermittel angesetzt wurde, bis zur Tropfnässe besprüht. Am folgenden Tag wurden die Pflanzen mit einer wäßrigen Sporensuspension von *Pyricularia oryzae* inokuliert. Anschließend wurden die Versuchspflanzen in Klimakammern bei 22 - 24°C und 95 - 99 % relativer Luftfeuchtigkeit für 6 Tage aufgestellt. Dann wurde das Ausmaß der Befallsentwicklung auf den Blättern visuell ermittelt.

| Wirkstoff aus Tabelle 1 | %-Befall der Blätter nach Applikation von 250 ppm-haltiger wäßriger Wirkstoffaufbereitung |
|---|---|
| | |
| Nr. 5 | 15 |
| Nr. 7 | 7 |
| Nr. 14 | 15 |
| Nr. 15 | 5 |
| | |
| Kontrolle (unbehandelt) | 85 |

### Beispiel 4

### Fungizide Wirkung

### Wirksamkeit gegen Plasmopara viticola

Blätter von Topfreben der Sorte "Müller-Thurgau" wurden mit wäßriger Wirkstoffauf-bereitung, die mit einer Stammlösung aus 10 % Wirkstoff, 63 % Cyclohexanon und 27 % Emulgiermittel angesetzt wurde, bis zur Tropfnäße besprüht. Um die Dauerwirkung der Substanzen beurteilen zu können, wurden die Pflanzen nach dem Antrocknen des Spritzbelages für 7 Tage im Gewächshaus aufgestellt. Erst dann wurden die Blätter mit einer wäßrigen Zoosporenaufschwemmung von *Plasmopara viticola* inokuliert. Danach wurden die Reben zunächst für 48 Stunden in einer wasserdampfgesättigten Kammer bei 24°C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

| Wirkstoff aus Tabelle 1 | %-Befall der Blätter nach Applikation von 250 ppm-haltiger wäßriger Wirkstoffaufbereitung |
|---|---|
| | |
| Nr. 2 | 0 |
| Nr. 14 | 0 |
| Nr. 18 | 10 |
| | |
| Kontrolle (unbehandelt) | 90 |

## Patentansprüche

1. Verwendung von Phenylessigsäureamiden der Formel I 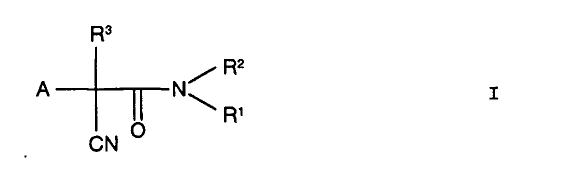 als Pflanzenschutzmittel mit herbizider Wirkung, wobei die Reste folgende Bedeutungen haben:
A Aryl, das durch folgende Gruppen ein- oder mehrfach substituiert sein kann: Wasserstoff, Halogen, Cyano, Nitro, Hydroxy, Mercapto, Thiocyanato, Carboxy, Amino, C₁-C₆-Alkyl, C₁-C₆-Alkoxy-C₁-C₆-Alkyl, C₁-C₆-Cyanoalkyl, C₂-C₆-Alkenyl, C₁-C₆-Alkoxy-C₂-C₆-Alkenyl, C₂-C₆-Alkinyl, C₁-C₆-Alkoxy-C₃-C₆-Alkinyl, C₃-C₆-Alkinyl-C₁₋C₆-Alkoxy, C₃-C₇-Cycloalkyl, C₁-C₆-Alkyl-C₃-C₇-Cycloalkyl, C₁-C₆-Alkoxy-C₃-C₇-Cycloalkyl, C₃-C₇-Cycloalkyloxy, C₃-C₇-Cyanocycloalkoxy, C₁-C₆-Alkyl-C₃-C₇-Cycloalkoxy, C₃-C₇-Halogencycloalkyl, C₃-C₇-Cyanocycloalkyl, C₃-C₇-Halogencycloalkoxy, C₅-C₇-Cycloalkenyl, C₁-C₆-Alkyl-C₅-C₇-Cycloalkenyl, C₁-C₆-Alkoxy-C₅-C₇-Cycloalkenyl, C₅-C₇-Cyanocycloalkenyl, C₅-C₇-Halogencycloalkenyl, C₁-C₆-Halogenalkyl, C₂-C₆-Halogenalkenyl, C₃-C₆-Halogenalkinyl, C₂-C₆-Cyanoalkenyl, C₃-C₆-Cyanoalkinyl, C₁-C₆-Alkoxy, C₁-C₆-Cyanoalkoxy, C₁-C₆-Halogenalkoxy, C₂-C₆-Alkenyloxy, C₂-C₆-Cyanalkenyloxy, C₂-C₆-Halogenalkenyloxy, C₂-C₆-Alkinyloxy, C₁-C₆-Alkylthio, C₁-C₆-Cyanoalkylthio, C₁-C₆-Halogenalkylthio, C₁-C₆-Alkoxy- C₁-C₆-Alkylthio, C₁-C₆-Alkoxycarbonyl, C₁-C₆-Alkylcarbonyl, C₁-C₆-Alkylcarboxy- C₁-C₆-Alkyl, C₁-C₆-Alkylcarbonyl-C₁-C₆-Alkyl, C₁-C₆-Alkylcarbonylamino, Arylcarbonylamino, Arylsulphinyl, Arylsulphonyl, Heteroarylsulphinyl, Heteroarylsulphonyl, Aryl-C₁-C₆-alkyl, Aryl-C₂-C₆-alkenyl, Aryl-C₃-C₆-alkinyl, Aryl-C₁-C₆-alkoxy, Aryl-C₂-C₆-alkenyloxy, Aryl-C₃-C₆-alkinyloxy, C₁-C₆-Alkylsulphinyl, C₁-C₆-Alkylsulphonyl, C₁-C₆-Alkylamino, Di-C₁-C₆-Alkylamino, Aryl, Aryloxy, Arylthio oder - C(R⁴)=NR⁵,
R¹ Aryl oder Aryl-C₁-C₆-alkyl, wobei der Arylrest jeweils durch folgende Gruppen ein- oder mehrfach substituiert sein kann: Wasserstoff, Halogen, Cyano, Nitro, Hydroxy, Mercapto, Thiocyanato, Carboxy, Amino, C₁-C₆-Alkyl, C₁-C₆-Alkoxy-C₁-C₆-Alkyl, C₁-C₆-Cyanoalkyl, C₂-C₆-Alkenyl, C₁-C₆-Alkoxy-C₂-C₆-Alkenyl, C₂-C₆-Alkinyl, C₁₋C₆-Alkoxy-C₃-C₆-Alkinyl, C₃-C₆-Alkinyl-C₁₋C₆-Alkoxy, C₃-C₇-Cycloalkyl, C₁-C₆-Alkyl-C₃-C₇-Cycloalkyl, C₁-C₆-Alkoxy-C₃-C₇-Cycloalkyl, C₃-C₇-Cycloalkyloxy, C₃-C₇-Cyanocycloalkoxy, C₁-C₆-Alkyl-C₃-C₇-Cycloalkoxy, C₃-C₇-Halogencycloalkyl, C₃-C₇-Cyanocycloalkyl, C₃-C₇-Halogencycloalkoxy, C₅-C₇-Cycloalkenyl, C₁-C₆-Alkyl-C₅-C₇-Cycloalkenyl, C₁-C₆-Alkoxy-C₅-C₇-Cycloalkenyl, C₅-C₇-Cyanocycloalkenyl, C₅-C₇-Halogencycloalkenyl, C₁-C₆-Halogenalkyl, C₂-C₆-Halogenalkenyl, C₃-C₆-Halogenalkinyl, C₂-C₆-Cyanoalkenyl, C₃-C₆-Cyanoalkinyl, C₁-C₆-Alkoxy, C₁-C₆-Cyanoalkoxy, C₁-C₆-Halogenalkoxy, C₂-C₆-Alkenyloxy, C₂-C₆-Cyanalkenyloxy, C₂-C₆-Halogenalkenyloxy, C₂-C₆-Alkinyloxy, C₁-C₆-Alkylthio, C₁-C₆-Cyanoalkylthio, C₁-C₆-Halogenalkylthio, C₁-C₆-Alkoxy-C₁-C₆--Alkylthio, C₁-C₆-Alkoxycarbonyl, C₁-C₆-Alkylcarbonyl, C₁-C₆-Alkylcarboxy-C₁-C₆-Alkyl, C₁-C₆-Alkylcarbonyl-C₁-C₆-Alkyl, C₁-C₆-Alkylcarbonylamino, Arylcarbonylamino, Arylsulphinyl, Arylsulphonyl, Heteroarylsulphinyl, Heteroarylsulphonyl, Aryl-C₁-C₆-Alkyl, Aryl-C₂-C₆-Alkenyl, Aryl-C₃-C₆-Alkinyl, Aryl-C₁-C₆-Alkoxy, Aryl-C₂-C₆-Alkenyloxy, Aryl-C₃-C₆-Alkinyloxy, C₁-C₆-Alkylsulphinyl, C₁-C₆-Alkylsulphonyl, C₁-C₆-Alkylamino, di-C₁-C₆-Alkylamino, Aryl, Aryloxy, Arylthio oder - C(R⁴)=NR⁵,
R², R³ Wasserstoff, C₁-C₆-Alkyl, C₂-C₆-Alkenyl, C₃-C₇-Cycloalkyl, C₁-C₆-Alkylaryl, welche ggf. teilweise oder vollständig halogeniert sein können oder einen bis drei Substituenten ausgewählt aus C₁-C₄-Alkoxy, C₁-C₄-Halogenalkoxy, C₁-C₄-Alkylthio, C₃-C₇-Cycloalkyl, C₅-C₇-Cycloalkenyl tragen können;
R⁴ Wasserstoff oder C₁-C₆-Alkyl,
R⁵ Hydroxy, C₁-C₄-Alkoxy, Aryl-C₁-C₄-Alkoxy, Aryloxy,
sowie deren landwirtschaftlich brauchbaren Salze.

2. Verwendung der Phenylessigsäureamide der Formel I als Pflanzenschutzmittel mit fungizider Wirkung zur Behandlung von Pilzen oder der vor Pilzbefall zu schützenden Saatgüter, Pflanzen oder den Erdboden.

3. Verwendung nach Anspruch 1 oder 2, wobei A unsubstituiertes und substituiertes Phenyl oder Naphthyl bedeutet.

4. Verwendung nach einem der Ansprüche 1 - 3, wobei R¹ unsubstituiertes und substituiertes Phenyl bedeutet.

5. Verwendung nach einem der Ansprüche 1 - 4, wobei R² Wasserstoff bedeutet.

6. Verwendung nach einem der Ansprüche 1 - 5, wobei R³ Wasserstoff; C₁-C₆-Alkyl, insbesondere Methyl, Ethyl, Propyl; C₃-C₇-Cycloalkyl, insbesondere Cyclopropyl bedeutet.

7. Verwendung nach einem der Ansprüche 1 - 6, wobei A oder R¹ substituiertes Phenyl bedeutet, der Phenylring ein-, zweioder dreifach substituiert ist durch Halogen, Alkyl, Alkoxy, Nitro, Cyano, Halogenalkyl oder Phenyl.

8. Verwendung nach Anspruch 7, wobei der Phenylring einfach substituiert ist durch Halogen, Alkyl, Alkoxy, Nitro, Halogenalkyl oder Cyano.

9. Verwendung nach Anspruch 8, wobei der Phenylring folgende Bedeutungen hat: 4-Halogenphenyl, 4-Alkylphenyl, 4-Alkoxyphenyl, 4-Nitrophenyl, 4-Cyanophenyl, 4-Trifluormethylphenyl, 2-Halogenphenyl, 2-Alkylphenyl, 2-Alkoxyphenyl, 2-Nitrophenyl, 2-Cyanophenyl, 2-Trifluormethylphenyl, 3-Halogenphenyl, 3-Alkylphenyl, 3-Alkoxyphenyl, 3-Nitrophenyl, 3-Cyanophenyl, 3-Trifluormethylphenyl.

10. Verwendung nach Anspruch 7, wobei der Phenylring zweifach substituiert ist durch Halogen oder Alkyl.

11. Verwendung nach Anspruch 10, wobei der Phenylring folgende Bedeutungen hat: 2,3-Dihalogenphenyl; 2,4-Dihalogenphenyl; 2,5-Dihalogenphenyl; 2,6-Dihalogenphenyl; 3,4-Dihalogenphenyl; 3,5-Dihalogenphenyl; 2,3-Dimethylphenyl; 2,4-Dimethylphenyl; 2,5-Dimethylphenyl; 2,6-Dimethylphenyl; 3,4-Dimethylphenyl oder 3,5-Dimethylphenyl.

12. Verfahren zur Behandlung von Pflanzen zur Bekämpfung von Schadpilzen; zur Bekämpfung von Schadpilzen; oder zur Bekämpfung unerwünschten Wuchses von Schadpflanzen, **dadurch gekennzeichnet, daß** man
i) die Pflanzen oder deren Lebensraum,
ii) die Pilze, deren Lebensraum oder die vor Pilzbefall zu schützenden Materialien, Pflanzen, Samen, Böden, Flächen oder Räume oder
iii) die Schadpflanzen oder deren Lebensraum
mit einer wirksamen Menge einer Verbindung der in Anspruch 1 angegebenen Formel I, worin die Reste die in einem der Ansprüche 1 bis 11 angegebene Bedeutung haben, oder einem diese Verbindung enthaltenden Mittel in Kontakt bringt, mit Ausnahme der Verbindungen, in denen A eine unsubstituierte Phenylgruppe; R³ Wasserstoff oder C₁- bis C₅-Alkyl bedeutet; und R¹ Aryl oder Aryl, das durch Halogen substituiert ist, oder Arylalkyl ist.

13. Pflanzenschutzmittel enthaltend im Gemisch mit Hilfsstoffen, die die Ausbringung des Mittels auf Freiflächen oder zur Behandlung von Pflanzen ermöglichen, mindestens eine der Verbindungen der in Anspruch 1 angegebenen Formel I, worin die Reste die in einem der Ansprüche 1 bis 11 angegebene Bedeutung haben, mit Ausnahme der Verbindungen, in denen A eine unsubstituierte Phenylgruppe; R³ Wasserstoff oder C₁- bis C₅-Alkyl bedeutet; und R¹ Aryl oder Aryl, das durch Halogen substituiert ist, oder Arylalkyl ist.

14. Verbindungen der in Anspruch 1 angegebenen Formel I, worin die Reste die in einem der Ansprüche 1 bis 11 angegebene Bedeutung haben,
mit Ausnahme der Verbindungen, in denen A eine unsubstituierte Phenylgruppe; R³ Wasserstoff oder C₁- bis C₅-Alkyl bedeutet; und R¹ Aryl oder Aryl ist, das durch Halogen substituiert ist, oder Arylalkyl ist,
mit Ausnahme der Verbindungen, in denen R³ Wasserstoff ist, R¹ Phenyl, Naphthyl oder Phenyl-C₁-C₄-alkyl ist und R² Wasserstoff, Alkyl, Alkenyl oder C₅-C₇-Cycloalkyl ist,
für den Fall, daß R³ Wasserstoff ist, A nicht 2-Nitrophenyl oder 2-Aminophenyl bedeutet; und
mit Ausnahme der folgenden Verbindungen:
2-Cyano-2-phenyl-hexansäure-(N-4-ethoxyphenyl)amid;
2-Cyano-2-phenyl-butansäure-(N-4-ethoxyphenyl)amid;
2-Cyano-2-phenyl-essigsäure-(N-2,4,6-trimethoxyphenyl)amid;
2-Cyano-2-(4-methoxyphenyl)essigsäure-(N-2,6-dimethylphenyl)amid
2-Cyano-2-phenyl-essigsäure-(N-2-methylphenyl)amid;
2-Cyano-2-phenyl-essigsäure-(N-3-methylphenyl)amid;
2-Cyano-2-phenyl-essigsäure-(N-4-methylphenyl)amid;
2-Cyano-2-(2-nitro-4-trifluormethylphenyl)essigsäure-N-phenylamid;
2-Cyano-2-(2-amino-4-trifluormethylphenyl)essigsäure-N-phenylamid.

15. Verbindung, ausgewählt unter:
2-Cyano-2-phenyl-essigsäure-(N-3-chlorphenyl)amid;
2-Cyano-2-phenyl-essigsäure-(N-2,5-dichlorphenyl)amid;
2-Cyano-2-phenyl-essigsäure-(N-2,6-dichlorphenyl)amid;
2-Cyano-2-phenyl-essigsäure-(N-3,5-dichlorphenyl)amid;
2-Cyano-2-phenyl-propionsäure-(N-3-chlorphenyl)amid;
2-Cyano-2-phenyl-essigsäure-(N-4-fluorphenyl)amid;
2-Cyano-2-phenyl-essigsäure-(N-2-fluorphenyl)amid;
2-Cyano-2-phenyl-essigsäure-(N-3-fluorphenyl)amid;
2-Cyano-2-phenyl-propionsäure-(N-4-fluorphenyl)amid;
2-Cyano-2-phenyl-propionsäure-(N-2-fluorphenyl)amid;
2-Cyano-2-phenyl-propionsäure-(N-3-fluorphenyl)amid.

## Claims

1. The use of phenylacetamides of the formula I 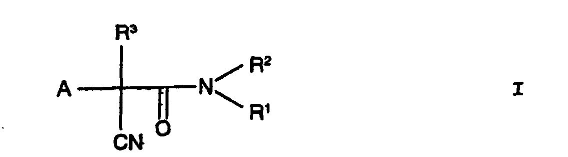 as crop protection agents having herbicidal action, where the radicals have the following meanings:
A is aryl which can be mono- or polysubstituted by the following groups: hydrogen, halogen, cyano, nitro, hydroxyl, mercapto, thiocyanato, carboxyl, amino, C₁-C₆-alkyl, C₁-C₆-alkoxy-C₁-C₆-alkyl, C₁-C₆-cyanoalkyl, C₂-C₆-alkenyl, C₁-C₆-alkoxy-C₂-C₆-alkenyl, C₂-C₆-alkynyl, C₁-C₆-alkoxy-C₃-C₆-alkynyl, C₃-C₆-alkynyl-C₁-C₆-alkoxy, C₃-C₇-cycloalkyl, C₁-C₆-alkyl-C₃-C₇-cycloalkyl, C₁-C₆-alkoxy-C₃-C₇-cycloalkyl, C₃-C₇-cycloalkyloxy, C₃-C₇-cyanocycloalkoxy, C₁-C₆-alkyl-C₃-C₇-cycloalkoxy, C₃-C₇-halocycloalkyl, C₃-C₇-cyanocycloalkyl, C₃-C₇-halocycloalkoxy, C₅-C₇-cycloalkenyl, C₁-C₆-alkyl-C₅-C₇-cycloalkenyl, C₁-C₆-alkoxy-C₅-C₇-cycloalkenyl, C₅-C₇-cyanocycloalkenyl, C₅-C₇-halocycloalkenyl, C₁-C₆-haloalkyl, C₂-C₆-haloalkenyl, C₃-C₆-haloalkynyl, C₂-C₆-cyanoalkenyl, C₃-C₆-cyanoalkynyl, C₁-C₆-alkoxy, C₁-C₆-cyanoalkoxy, C₁-C₆-haloalkoxy, C₂-C₆-alkenyloxy, C₂-C₆-cyanoalkenyloxy, C₂-C₆-haloalkenyloxy, C₂-C₆-alkynyloxy, C₁-C₆-alkylthio, C₁-C₆-cyanoalkylthio, C₁-C₆-haloalkylthio, C₁-C₆-alkoxy-C₁-C₆-alkylthio, C₁-C₆-alkoxycarbonyl, C₁-C₆-alkylcarbonyl, C₁-C₆-alkylcarboxyl-C₁-C₆-alkyl, C₁-C₆-alkylcarbonyl-C₁-C₆-alkyl, C₁-C₆-alkylcarbonylamino arylcarbonylamino, arylsulfinyl, arylsulfonyl, heteroarylsulfinyl, heteroarylsulfonyl, aryl-C₁-C₆-alkyl, aryl-C₂-C₆-alkenyl, aryl-C₃-C₆-alkynyl, aryl-C₁-C₆-alkoxy, aryl-C₂-C₆-alkenyloxy, aryl-C₃-C₆-alkynyloxy, C₁-C₆-alkylsulfinyl, C₁-C₆-alkylsulfonyl, C₁-C₆-alkylamino, di-C₁-C₆-alkylamino, aryl, aryloxy, arylthio or -C(R⁴)=NR⁵,
R¹ is aryl or aryl-C₁-C₆-alkyl, where the aryl radical can in each case be mono- or polysubstituted by the following groups: hydrogen, halogen, cyano, nitro, hydroxyl, mercapto, thiocyanato, carboxyl, amino, C₁-C₆-alkyl, C₁-C₆-alkoxy-C₁-C₆-alkyl, C₁-C₆-cyanoalkyl, C₂-C₆-alkenyl, C₁-C₆-alkoxy-C₂-C₆-alkenyl, C₂-C₆-alkynyl, C₁-C₆-alkoxy-C₃-C₆-alkynyl, C₃-C₆-alkynyl-C₁-C₆-alkoxy, C₃-C₇-cycloalkyl, C₁-C₆-alkyl-C₃-C₇-cycloalkyl, C₁-C₆-alkoxy-C₃-C₇-cycloalkyl, C₃-C₇-cycloalkyloxy, C₃-C₇-cyanocycloalkoxy, C₁-C₆-alkyl-C₃-C₇-cycloalkoxy, C₃-C₇-halocycloalkyl, C₃-C₇-cyanocycloalkyl, C₃-C₇-halocycloalkoxy, C₅-C₇-cycloalkenyl, C₁-C₆-alkyl-C₅-C₇-cycloalkenyl, C₁-C₆-alkoxy-C₅-C₇-cycloalkenyl, C₅-C₇-cyanocycloalkenyl, C₅-C₇-halocycloalkenyl, C₁-C₆-haloalkyl, C₂-C₆-haloalkenyl, C₃-C₆-haloalkynyl, C₂-C₆-cyanoalkenyl, C₃-C₆-cyanoalkynyl, C₁-C₆-alkoxy, C₁-C₆-cyanoalkoxy, C₁-C₆-haloalkoxy, C₂-C₆-alkenyloxy, C₂-C₆-cyanoalkenyloxy, C₂-C₆-haloalkenyloxy, C₂-C₆-alkynyloxy, C₁-C₆-alkylthio, C₁-C₆-cyanoalkylthio, C₁-C₆-haloalkylthio, C₁-C₆-alkoxy-C₁-C₆-alkylthio, C₁-C₆-alkoxycarbonyl, C₁-C₆-alkylcarbonyl, C₁-C₆-alkylcarboxyl-C₁-C₆-alkyl, C₁-C₆-alkylcarbonyl-C₁-C₆-alkyl, C₁-C₆-alkylcarbonylamino, arylcarbonylamino, arylsulfinyl, arylsulfonyl, heteroarylsulfinyl, heteroarylsulfonyl, aryl-C₁-C₆-alkyl, aryl-C₂-C₆-alkenyl, aryl-C₃-C₆-alkynyl, aryl-C₁-C₆-alkoxy, aryl-C₂-C₆-alkenyloxy, aryl-C₃-C₆-alkynyloxy, C₁-C₆-alkylsulfinyl, C₁-C₆-alkylsulfonyl, C₁-C₆-alkylamino, di-C₁-C₆-alkylamino, aryl, aryloxy, arylthio or -C(R⁴)=NR⁵,
R², R³ are hydrogen, C₁-C₆-alkyl, C₂-C₆-alkenyl, C₃-C₇-cycloalkyl, C₁-C₆-alkylaryl, which may be unsubstituted or partially or fully halogenated or may carry one to three substituents selected from the group consisting of C₁-C₄-alkoxy, C₁-C₄-haloalkoxy, C₁-C₄-alkylthio, C₃-C₇-cycloalkyl, C₅-C₇-cycloalkenyl;
R⁴ is hydrogen or C₁-C₆-alkyl,
R⁵ is hydroxyl, C₁-C₄-alkoxy, aryl-C₁-C₄-alkoxy, aryloxy,
and their agriculturally useful salts.

2. The use of the phenyl acetamides of the formula I as crop protection agents having fungicidal action for treating fungi or seeds, plants or the soil to be protected from fungal attack.

3. The use as claimed in claim 1 or 2, where A is unsubstituted or substituted phenyl or naphthyl.

4. The use as claimed in any of claims 1 - 3, where R¹ is unsubstituted or substituted phenyl.

5. The use as claimed in any of claims 1 - 4, where R² is hydrogen.

6. The use as claimed in any of claims 1 - 5, where R³ is hydrogen; C₁-C₆-alkyl, in particular methyl, ethyl, propyl; C₃-C₇-cycloalkyl, in particular cyclopropyl.

7. The use as claimed in any of claims 1 - 6, where A or R¹ is substituted phenyl, the phenyl ring being mono-, di- or trisubstituted by halogen, alkyl, alkoxy, nitro, cyano, haloalkyl or phenyl.

8. The use as claimed in claim 7, where the phenyl ring is monosubstituted by halogen, alkyl, alkoxy, nitro, haloalkyl or cyano.

9. The use as claimed in claim 8, where the phenyl ring has the following meanings: 4-halophenyl, 4-alkylphenyl, 4-alkoxyphenyl, 4-nitrophenyl, 4-cyanophenyl, 4-trifluoromethylphenyl, 2-halophenyl, 2-alkylphenyl, 2-alkoxyphenyl, 2-nitrophenyl, 2-cyanophenyl, 2-trifluoromethylphenyl, 3-halophenyl, 3-alkylphenyl, 3-alkoxyphenyl, 3-nitrophenyl, 3-cyanophenyl, 3-trifluoromethylphenyl.

10. The use as claimed in claim 7, where the phenyl ring is disubstituted by halogen or alkyl.

11. The use as claimed in claim 10, where the phenyl ring has the following meanings: 2,3-dihalophenyl; 2,4-dihalophenyl; 2,5-dihalophenyl; 2,6-dihalophenyl; 3,4-dihalophenyl; 3,5-dihalophenyl; 2,3-dimethylphenyl; 2,4-dimethylphenyl; 2,5-dimethylphenyl; 2,6-dimethylphenyl; 3,4-dimethylphenyl or 3,5-dimethylphenyl.

12. A method for treating plants for controlling harmful fungi; for controlling harmful fungi; or for controlling undesirable growth of harmful plants, which comprises bringing
i) the plants or their habitat
ii) the fungi, their habitat or materials, plants, seeds, soils, areas or spaces to be protected against fungal attack, or
iii) the harmful plants or their habitat
into contact with an effective amount of a compound of the formula I given in claim 1, where the radicals are as defined. in any of claims 1 to 11, or with a composition comprising this compound, except for the compounds in which A is an unsubstituted phenyl group; R³ is hydrogen or C₁ to C₅-alkyl; and R¹ is aryl, aryl which is substituted by halogen, or arylalkyl.

13. A crop protection composition, comprising, in a mixture with auxiliaries which permit the composition to be applied to areas free from crops or for the treatment of plants, at least one of the compounds of the formula I given in claim 1, where the radicals are as defined in any of claims 1 to 11, except for the compounds in which A is an unsubstituted phenyl group; R³ is hydrogen or C₁ to C₅-alkyl; and R¹ is aryl, aryl which is substituted by halogen, or arylalkyl.

14. A compound of the formula I given in claim 1, where the radicals are as defined in any of claims 1 to 11,
except for the compounds in which A is an unsubstituted phenyl group; R³ is hydrogen or C₁- to C₅-alkyl; and R¹ is aryl, aryl which is substituted by halogen, or arylalkyl;
except for the compounds in which R³ is hydrogen, R¹ is phenyl, naphthyl or phenyl-C₁-C₄-alkyl and R² is hydrogen, alkyl, alkenyl or C₅-C₇-cycloalkyl;
where, if R³ is hydrogen, A is not 2-nitrophenyl or 2-aminophenyl; and
except for the following compounds:
N-(4-ethoxyphenyl)-2-cyano-2-phenyl hexamide;
N-(4-ethoxyphenyl)-2-cyano-2-phenylbutamide;
N-(2,4,6-trimethoxyphenyl)-2-cyano-2-phenylacetamide;
N-(2,6-dimethylphenyl)-2-cyano-2-(4-methoxyphenyl)acetamide;
N-(2-methylphenyl)-2-cyano-2-phenylacetamide;
N-(3-methylphenyl)-2-cyano-2-phenylacetamide;
N-(4-methylphenyl)-2-cyano-2-phenylacetamide;
N-(phenyl)-2-cyano-2-(2-nitro-4-trifluoromethylphenyl)-acetamide;
N-(phenyl)-2-cyano-2-(2-amino-4-trifluoromethylphenyl)-acetamide.

15. A compound, selected from:
N-(3-chlorophenyl)-2-cyano-2-phenylacetamide;
N-(2,5-dichiorophenyl)-2-cyano-2-phenylacetamide;
N-(2,6-dichlorophenyl)-2-cyano-2-phenylacetamide;
N-(3,5-dichlorophenyl)-2-cyano-2-phenylacetamide
N-(3-chlorophenyl)-2-cyano-2-phenylpropionamide;
N-(4-fluorophenyl)-2-cyano-2-phenylacetamide;
N-(2-fluorophenyl)-2-cyano-2-phenylacetamide;
N-(3-fluorophenyl)-2-cyano-2-phenylacetamide;
N-(4-fluorophenyl)-2-cyano-2-phenylpropionamide;
N-(2-fluorophenyl)-2-cyano-2-phenylpropionamide;
N-(3-fluorophenyl)-2-cyano-2-phenylpropionamide.

## Revendications

1. Utilisation des phénylacétamides de formule I 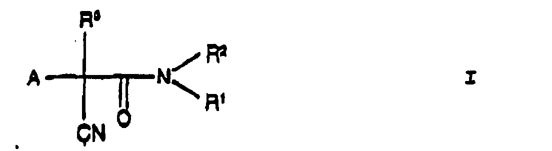 en tant que produits phytosanitaires à activité herbicide, les symboles de la formule ayant les significations suivantes :
A : un radical aryle qui peut porter un ou plusieurs des substituants suivants : l'hydrogène, des halogènes, des groupes cyano, nitro, hydroxy, mercapto, thiocyanato, carboxy, amino, alkyle en C1-C6, (alcoxy en C1-C6)alkyle en C1-C6, cyano (alkyle en C1-C6), alcényle en C2-C6, (alcoxy en C1-C6)alcényle en C2-C6, aloynyle en C2-C6, (alcoxy en C1-C6)alcynyle en C3-C6, (alcynyle en C3-C6)alcoxy en C1-C6, cycloalkyle en C3-C7,(alkyle en C1-C6)-cycloalkyle en C3-C7, (alcoxy en C1-C6)cycloalkyle en C3-C7, cycloalcoxy en C3-C7, cyano(cycloalcoxy en C3-C7), (alkyle en C1-C6)cycloalcoxy en C3-C7, halogénocycloalkyle en C3-C7, cyano(cycloalkyle en C3-C7), halogénocycloalcoxy en C3-C7, cycloalcényle en C5-C7, (alkyle en C1-C6)cycloalcényle en C5-C7, (alcoxy en C1-C6)cycloalcényle en C5-C7, cyano(cycloaloényle en C5-C7), halogénocycloalcényle en C5-C7, halogénoalkyle en C1-C6, halogénoalcényle en C2-C6, halogénoalcynyle en C3-C6, cyano(aicényle en C2-C6), cyano(alcynyle en C3-C6), alcoxy en C1-C6, cyano(alcoxy en C1-C6), halogénoalcoxy en C1-C6, alcényloxy en C2-C6, cyano(alcényloxy en C2-C6), halogénoalcényloxy en C2-C6, alcynyloxy en C2-C6, alkylthio en C1-C6, cyano(alkylthio en C1-C6), halogénoalkylthio en C1-C6, (alcoxy en C1-C6)alkylthio en C1-C6), (alcoxy en C1-C6)carbonyle, (alkyle en C1-C6)carbonyle, (alkyle en C 1-C6)carboxy-alkyle en C1-C6, (alkyle en C1-C6)carbonyl-alkyle en C1-C6, (alkyle en C1-C6)carbonylamino, arylcarbonylamino, arylsulfinyle, arylsulfonyle, hétéroarylsulfinyle, hétéroarylsulfonyle, aryl-alkyle en C1-C6, aryl-alcényle en C2-C6, aryl-alcynyle en C3-C6, aryl-alcoxy en C1-C6, aryl-alcényloxy en C2-C6, aryl-alcynyloxy en C3-C6, alkylsulfinyle en C1-C6, alkylsulfonyle en C1-C6, alkylamino en C1-C6, di-(alkyle en C1-C6)amino, aryle, aryloxy, arylthio ou -C(R⁴)=NR⁵,
R¹ : un radical aryle ou aryl-alkyle en C1-C6, la partie aryle pouvant porter un ou plusieurs des substituants suivants : l'hydrogène, des halogènes, des groupes cyano, nitro, hydroxy, mercapto, thiocyanato, carboxy, amino, alkyle en C1-C6, (alcoxy en C1-C6)alkyle en C1-C6, cyano(alkyle en C1-C6), alcényle en C2-C6, (alcoxy en C1-C6)alcényle en C2-C6, alcynyle en C2-C6, (alcoxy en C1-C6)alcynyle en C3-C6, (alcynyle en C3-C6)alcoxy en C1-C6, cycloalkyle en C3-C7, (alkyle en C1-C6)cycloalkyle en C3-C7, (alcoxy en C1-C6)-cycloalkyle en C3-C7, cycloalcoxy en C3-C7, cyano(cycloalcoxy en C3-C7), (alkyle en C1-C6)-cycloalcoxy en C3-C7, halogénocycloalkyle en C3-C7, cyano(cycloalkyle en C3-C7), halogénooycloalcoxy en C3-C7, cycloalcényle en C5-C7, (alkyle en C1-C6)cycloalcényle en C5-C7, (alcoxy en C1-C6)cycloalcényle en C5-C7, cyano(cycloalcényle en C5-C7), halogénocycloalcényle en C5-C7, halogénoalkyle en C1-C6, halogénoalcényle en C2-C6, halogénoalcynyle en C3-C6, cyano(alcényle en C2-C6), cyano(alcynyle en C3-C6), alcoxy en C1-C6, cyano(alcoxy en C1-C6), halogénoalcoxy en C1-C6, alcényloxy en C2-C6, cyano(alcényloxy en C2-C6), halogénoalcényloxy en C2-C6, alcynyloxy en C2-C6, alkylthio en C1-C6, cyano(alkylthio en C1-C6), halogénoalkylthio en C1-C6, (alcoxy en C1-C6)alkylthio en C1-C6, (alcoxy en C1-C6)carbonyle, (alkyle en C1-C6)carbonyle, (alkyle en C1-C6)carboxy-alkyle en C1-C6, (alkyle en Cl-C6)-carbonyl-alkyle en C1-C6, (alkyle en C1-C6)carbonylamino, arylcarbonylamino, arylsulfinyle, arylsulfonyle, hétéroarylsulfinyle, hétéroarylsulfonyle, aryl-alkyle en C1-C6, aryl-alcényle en C2-C6, aryl-alcynyle en C3-C6, aryl-alcoxy en C1-C6, aryl-alcényloxy en C2-C6, aryl-alcynyloxy en C3-C6, alkylsulfinyle en C1-C6, alkylsulfonyle en C1-C6, alkylamino en C1-C6, di-(alkyle en C1-C6)amino, aryle, aryloxy, arylthio ou -C(R⁴)=NR⁵,
R², R³ ; l'hydrogène, des groupes alkyle en C1-C6, alcényle en C2-C6, cycloalkyle en C3-C7, (alkyle en C1-C6)aryle, qui peuvent être halogénés en totalité ou en partie ou peuvent porter un à trois substituants choisis parmi les groupes alcoxy en C1-C4, halogénoalcoxy en C1-C4, alkylthio en C1-C4, cycloalkyle en C3-C7, cycloalcényle en C5-C7 ;
R⁴ ; l'hydrogène ou un groupe alkyle en C1-C6,
R⁵ : un groupe hydroxy, alcoxy en C1-C4, aryl-alcoxy en C1-C4, aryloxy, et leurs sels aptes aux applications agricoles.

2. Utilisation des phénylacétamides de formule I en tant que produits phytosanitaires à activité fongicide pour combattre les mycètes ou protéger les semences, les végétaux ou le sol contre une attaque par les mycètes.

3. Utilisation selon la revendication 1 ou 2, dans laquelle A représente un groupe phényle ou naphtyle substitué ou non.

4. Utilisation selon l'une des revendications 1 à 3, dans laquelle R¹ représente un groupe phényle substitué ou non.

5. Utilisation selon l'une des revendications 1 à 4, dans laquelle R² représente l'hydrogène.

6. Utilisation selon l'une des revendications 1 à 5, dans laquelle R³ représente l'hydrogène, un groupe alkyle en C1-C6, plus spécialement méthyle, éthyle, propyle ; un groupe cycloalkyle en C3-C7, plus spécialement cyclopropyle.

7. Utilisation selon l'une des revendications 1 à 6, dans laquelle A ou R¹ représente un groupe phényle substitué, le cycle phényle portant un, deux ou trois substituants choisis parmi les halogènes, les groupes alkyle, alcoxy, nitre, cyano, halogénoalkyle et phényle.

8. Utilisation selon la revendication 7, dans laquelle le cycle phényle porte un substituant halogéno, alkyle, alcoxy, nitro, halogénoalkyle ou cyano.

9. Utilisation selon la revendication 8, dans laquelle le cycle phényle est l'un des suivants : 4-halogénophényle, 4-alkylphényle, 4-alcoxyphényle, 4-nitrophényle, 4-cyanophényle, 4-trifluorométhylphényle, 2-halogénophényle, 2-alkylphényle, 2-alcoxyphényle, 2-nitrophényle, 2-cyanophényle, 2-trifluorométhylphényle, 3-halogénophényle, 3-alkylphényle, 3-alcoxyphényle, 3-nitrophényle, 3-cyanophényle, 3-trifluorométhylphényle.

10. Utilisation selon la revendication 7, dans laquelle le cycle phényle porte deux substituants choisis parmi les halogènes et les groupes alkyle.

11. Utilisation selon la revendication 10, dans laquelle le cycle phényle est l'un des suivants : 2,3-dihalogénophényle; 2,4-dihalogénophényle ; 2,5-dihalogénophényle ; 2,6-dihalogénophényle ; 3,4-dihalogénophényle ; 3,5-dihalogénophényle ; 2,3-diméthylphényle ; 2,4-diméthylphényle ; 2,5-diméthylphényle ; 2,6-diméthylphényle ; 3,4-diméthylphényle ou 3,5-diméthylphényle.

12. Procédé pour traiter les végétaux en vue de combattre les mycètes, nuisibles ; pour combattre les mycètes nuisibles ; ou pour combattre les croissances indésirables de végétaux nuisibles, **caractérisé par le fait que**
i) on traite les végétaux ou leur habitat,
ii) on traite les mycètes, leur habitat ou les matériaux, végétaux, semences, sols, aires ou locaux qu'on veut protéger contre l'attaque par les mycètes, ou bien
iii) on traite les végétaux nuisibles ou leur habitat
par une quantité efficace d'un composé de formule I selon la revendication 1, dans laquelle les symboles ont les significations indiquées dans l'une des revendications 1 à 11, ou bien on les met en contact avec un produit contenant l'un de ces composés, à l'exception des composés pour lesquels A représente un groupe phényle non substitué ; R³ représente l'hydrogène ou un groupe alkyle en C1-C5 ; et R¹ représente un groupe aryle ou aryle substitué par des halogènes ou un groupe arylalkyle.

13. Produit phytosanitaire contenant, en mélange avec des produits auxiliaires qui permettent d'appliquer le produit sur les aires libres ou de traiter des végétaux, au moins un des composés de formule 1 selon la revendication 1 dans laquelle les symboles ont les significations indiquées dans l'une des revendications 1 à 11, à l'exception des composés pour lesquels A représente un groupe phényle non substitué ; R³ représente l'hydrogène ou un groupe alkyle en C1-C5 ; et R¹ représente un groupe aryle ou aryle substitué par des halogènes ou un groupe arylalkyle.

14. Composés de formule 1 selon la revendication 1, dans laquelle les symboles ont les significations indiquées dans l'une des revendications 1 à 11,
à l'exception des composés pour lesquels A représente un groupe phényle non substitué ; R³ représente l'hydrogène ou un groupe alkyle en C1-C5 ; et R¹ représente un groupe aryle ou aryle substitué par des halogènes ou un groupe arylalkyle,
à l'exception des composés pour lesquels R³ représente l'hydrogène, R¹ un groupe phényle, naphtyle ou phényl-alkyle en C1-C4 et R² l'hydrogène, un groupe alkyte, alcényle ou cycloalkyle en C5-C7,
et dans le cas où R³ représente l'hydrogène, A ne peut représenter un groupe 2-nitrophényle ou 2-aminophényle ; et
à l'exception des composés suivants :
2-cyano-2-phényl-hexano-(N-4-éthoxyphényl)amide ;
2-cyano-2-phényl-butano-(N-4-éthoxyphényl)amide ;
2-cyano-2-phényl-acéto-(N-2,4,6-triméthoxyphényl)amide ;
2-cyano-2-(4-méthoxyphényl)acéto-(N-2,6-diméthylphényl)amide ;
2-cyano-2-phényl-acéto-(N-2-méthylphényl)amide ;
2-cyano-2-phényl-acéto-(N-3-méthylphényl)amide ;
2-cyano-2-phényl-acéto-(N-4-méthylphényl)amide ;
2-cyano-2-(2-nitro-4-trifluorométhylphényl)acéto-N-phénylamide ;
2-cyano-2-(2-amino-4-trifluorométhylphényl)acéto-N-phénylamide.

15. Composé choisi parmi les suivants :
2-cyano-2-phényl-acéto-(N-3-chlorophényl)amide ;
2-cyano-2-phényl-acéto-(N-2,5-dichlorophényl)amide ;
2-cyano-2-phényl-acéto-(N-2,6-dichlorophényl)amide ;
2-cyano-2-phényl-acéto-(N-3,5-dichlorophényl)amide ;
2-cyano-2-phényl-propio-(N-3-chlorophényl)amide ;
2-cyano-2-phényl-acéto-(N-4-fluorophényl)amide ;
2-cyano-2-phényl-acéto-(N-2-fluorophényl)amide ;
2-cyano-2-phényl-acéto-(N-3-fluorophényl)amide ;
2-cyano-2-phényl-propio-(N-4-fluorophényl)amide ;
2-cyano-2-phényl-propio-(N2-2-fluorophényl)amide ;
2-cyano-2-phényl-propio-(N-3-fluorophényl)amide.
